(19) Europäisches Patentamt
European Patent Office
Office européen des brevets

(11) EP 1 644 520 B1

(12) **EUROPEAN PATENT SPECIFICATION**

(45) Date of publication and mention of the grant of the patent:
**19.09.2007 Bulletin 2007/38**

(21) Application number: **04738977.0**

(22) Date of filing: **05.07.2004**

(51) Int Cl.:
***C12Q 1/68*** *(2006.01)*

(86) International application number:
**PCT/DK2004/000480**

(87) International publication number:
**WO 2005/003384 (13.01.2005 Gazette 2005/02)**

# (54) METHOD FOR SELECTIVE DETECTION OF A TARGET NUCLEIC ACID

VERFAHREN ZUR SELEKTIVEN DETEKTION EINER ZIELNUKLEINSÄURE

PROCEDE DE DETECTION SELECTIVE D'UN ACIDE NUCLEIQUE CIBLE

(84) Designated Contracting States:
**AT BE BG CH CY CZ DE DK EE ES FI FR GB GR HU IE IT LI LU MC NL PL PT RO SE SI SK TR**

(30) Priority: **03.07.2003 DK 200301018**
**03.07.2003 US 484926 P**

(43) Date of publication of application:
**12.04.2006 Bulletin 2006/15**

(73) Proprietor: **Danmarks og Grønlands Geologiske Undersøgelse**
**1350 Copenhagen K (DK)**

(72) Inventors:
- **BENDER, Mikkel**
  **DK-2100 Copenhagen (DK)**
- **JACOBSEN, Carsten, Suhr**
  **DK-2700 Bronshoj (DK)**

(74) Representative: **Plougmann & Vingtoft A/S**
**Sundkrogsgade 9,**
**P.O. Box 831**
**2100 Copenhagen Ø (DK)**

(56) References cited:
**WO-A-01/20041** **WO-A-98/24933**
**WO-A-03/020902** **US-A- 5 738 993**
**US-A- 5 849 497**

- **GOOD ET AL: "Antisense inhibition of gene expression in bacteria by PNA targeted to mRNA" NATURE BIOTECHNOLOGY, NATURE PUBLISHING, US, vol. 16, April 1998 (1998-04), pages 355-358, XP002140166 ISSN: 1087-0156**
- **OERUM H: "PCR CLAMPING" CURRENT ISSUES IN MOLECULAR BIOLOGY, CAISTER ACADEMIC, WYMONDHAM, GB, vol. 2, no. 1, 2000, pages 27-30, XP008014249 ISSN: 1467-3037**
- **NIELSEN P E ET AL: "SEQUENCE-SPECIFIC TRANSCRIPTION ARREST BY PEPTIDE NUCLEIC ACID BOUND TO THE DNA TEMPLATE STRAND" GENE, ELSEVIER BIOMEDICAL PRESS. AMSTERDAM, NL, vol. 149, 1994, pages 139-145, XP002022886 ISSN: 0378-1119**
- **KYGER ERICH M ET AL: "Detection of hereditary hemochromatosis gene mutation by real-time fluorescence polymerase chain reaction and peptide nucleic acid clamping" ANALYTICAL BIOCHEMISTRY, vol. 260, no. 2, 1 July 1998 (1998-07-01), pages 142-148, XP002303784 ISSN: 0003-2697**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 644 520 B1

SEQUENCE LISTING

<110> Danmarks og Grønlands Geologiske Undersøgelse
Bender, Mikkel
Jacobsen, Carsten Suhr

< 120> Method for selective detection of a

target nucleic acid

<130> 33211PC01

<140> PCT/DK2004/0004480
<141> 2004-07-02

<150> PA 2003 01018
<151> 2004-07-03

<150> 60/484,926
<151> 2004-07-03

<160> 161

<170> FastSEQ for Windows Version 4.0

<210> 1
<211> 17
<212> DNA
<213> Artificial Sequence

<220>
<223> Salmonella 16S primer

<400> 1
tattaaccac aacacct 17

<210> 2
<211> 18
<212> DNA
<213> Artificial Sequence

<220>
<223> Salmonella 16S probe

<400> 2
aattgctgcg gttattaa 18

<210> 3
<211> 17
<212> DNA
<213> Artificial Sequence

<220>
<223> Yertsenia sp. 16S primer

<400> 3
ccgatggcgt gaggccc 17

<210> 4
<211> 15
<212> DNA
<213> Artificial Sequence

<220>
<223> Yertsenia sp. 16S probe

<400> 4
ctgggcacat ccgat 15

<210> 5
<211> 18
<212> DNA
<213> Artificial Sequence

<220>
<223> Camphylobacter sp. 16S primer

<400> 5
cattgtagca cgtgtgtc 18

<210> 6
<211> 20
<212> DNA
<213> Artificial Sequence

<220>
<223> Camphylobacter sp. 16S probe

<400> 6
ttgtatatgc cattgtagca 20

<210> 7
<211> 21
<212> DNA
<213> Artificial Sequence

<220>
<223> Clostridium perfringens 16S primer

<400> 7
gggtaccgtc attatcttcc c 21

<210> 8
<211> 18
<212> DNA
<213> Artificial Sequence

<220>
<223> Clostridium perfringens 16S probe

<400> 8
ggcttcctcc ttgggtac 18

<210> 9
<211> 21
<212> DNA
<213> Artificial Sequence

<220>
<223> Legionella sp.16S primer

<400> 9
ccaacagywa gttgacatcg t 21

<210> 10
<211> 21
<212> DNA
<213> Artificial Sequence

<220>
<223> Legionella sp.16S probe

<400> 10
tttcatataa ccaacagywa g 21

<210> 11
<211> 19
<212> DNA
<213> Artificial Sequence

<220>
<223> Listeria monocytogenes 16S primer

<400> 11
atagttttat gggattagc 19

<210> 12
<211> 17
<212> DNA
<213> Artificial Sequence

<220>
<223> Listeria monocytogenes 16S probe

<400> 12
actgagaata gttttat 17

<210> 13
<211> 24
<212> DNA
<213> Artificial Sequence

<220>
<223> Mycobacterium Sp. 16S primer

<400> 13
gcccgtatcg cccgcacgct caca 24

<210> 14
<211> 18
<212> DNA
<213> Artificial Sequence

<220>
<223> Mycobacterium Sp. 16S probe

<400> 14
ctctagtctg cccgtatc 18

<210> 15
<211> 15
<212> DNA

<213> Artificial Sequence

<220>
<223> Vibrio vulnificus 23S primer

<400> 15
tacttgtaac ccatc 15

<210> 16
<211> 12
<212> DNA
<213> Artificial Sequence

<220>
<223> Vibrio vulnificus 23S probe

<400> 16
aagatacttg ta 12

<210> 17
<211> 12
<212> DNA
<213> Artificial Sequence

<220>
<223> Universal Bacterial 16S primer

<400> 17
gctggcacgg ag 12

<210> 18
<211> 11
<212> DNA
<213> Artificial Sequence

<220>
<223> Universal Bacterial 16S probe

<400> 18
gcggctgctg g 11

<210> 19
<211> 13
<212> DNA
<213> Artificial Sequence

<220>
<223> Primer based on primer BSR1541/20 (ribosomal database project)

<400> 19
tgatccagcc gca 13

<210> 20
<211> 10
<212> DNA
<213> Artificial Sequence

<220>

<223> Probe based on primer BSR1541/20 (ribosomal database project)

<400> 20
gaggtgatcc 10

<210> 21
<211> 11
<212> DNA
<213> Artificial Sequence

<220>
<223> Primer based on primer BSR1407/16 (ribosomal database project)

<400> 21
gcggtgtgtr c 11

<210> 22
<211> 10
<212> DNA
<213> Artificial Sequence

<220>
<223> Probe based on primer BSR1407/16 (ribosomal database project)

<400> 22
gacgggcggt 10

<210> 23
<211> 12
<212> DNA
<213> Artificial Sequence

<220>
<223> Primer based on primer BSR1114/16 (ribosomal database project)

<400> 23
tgcgctcgtt rc 12

<210> 24
<211> 9
<212> DNA
<213> Artificial Sequence

<220>
<223> Probe based on primer BSR1114/16 (ribosomal database project)

<400> 24
gggttgcgc 9

<210> 25
<211> 12
<212> DNA
<213> Artificial Sequence

<220>
<223> Primer based on primer BSR798/15 (ribosomal database project)

<400> 25

gtatctaatc cc 12

<210> 26
<211> 10
<212> DNA
<213> Artificial Sequence

<220>
<223> Probe based on primer BSR798/15 (ribosomal database project)

<400> 26
ggggtatcta 10

<210> 27
<211> 13
<212> DNA
<213> Artificial Sequence

<220>
<223> Primer based on primer BSR65/17 (ribosomal database project)

<400> 27
cttgcatgtr tta 13

<210> 28
<211> 12
<212> DNA
<213> Artificial Sequence

<220>
<223> Probe based on primer BSR65/17 (ribosomal database project)

<400> 28
tcgacttgca tg 12

<210> 29
<211> 12
<212> DNA
<213> Artificial Sequence

<220>
<223> Primer based on primer BSR357/15 (ribosomal database project)

<400> 29
ctgcctyccg ta 12

<210> 30
<211> 9
<212> DNA
<213> Artificial Sequence

<220>
<223> Probe based on primer BSR357/15 (ribosomal database project)

<400> 30
ctgctgcct 9

<210> 31

<211> 14
<212> DNA
<213> Artificial Sequence

<220>
<223> Primer based on primer BSR534/18 (ribosomal database project)

<400> 31
ccgcggctgc tggc 14

<210> 32
<211> 9
<212> DNA
<213> Artificial Sequence

<220>
<223> Probe based on primer BSR534/18 (ribosomal database project)

<400> 32
attaccgcg 9

<210> 33
<211> 13
<212> DNA
<213> Artificial Sequence

<220>
<223> Primer based on primer NSR1787/18 (ribosomal database project)

<400> 33
ggttcaccta crg 13

<210> 34
<211> 12
<212> DNA
<213> Artificial Sequence

<220>
<223> Probe based on primer NSR1787/18 (ribosomal database project)

<400> 34
cygcaggttc ac 12

<210> 35
<211> 12
<212> DNA
<213> Artificial Sequence

<220>
<223> Primer based on primer NSR1642/16 (ribosomal database project)

<400> 35
ggcggtgtgt rc 12

<210> 36
<211> 8
<212> DNA
<213> Artificial Sequence

<220>
<223> Probe based on primer NSR1642/16 (ribosomal database project)

<400> 36
gacgggcg 8

<210> 37
<211> 16
<212> DNA
<213> Artificial Sequence

<220>
<223> Primer based on primer NSR1147/20 (ribosomal database project)

<400> 37
caattyyttt ragttt 16

<210> 38
<211> 11
<212> DNA
<213> Artificial Sequence

<220>
<223> Probe based on primer NSR1147/20 (ribosomal database project)

<400> 38
ccgtcaatty y 11

<210> 39
<211> 16
<212> DNA
<213> Artificial Sequence

<220>
<223> Primer based on primer NSR1438/20 (ribosomal database project)

<400> 39
atcacagacc tgttat 16

<210> 40
<211> 10
<212> DNA
<213> Artificial Sequence

<220>
<223> Probe based on primer NSR1438/20 (ribosomal database project)

<400> 40
gggcatcaca 10

<210> 41
<211> 13
<212> DNA
<213> Artificial Sequence

<220>
<223> Primer based on primer NSR980/18 (ribosomal database project)

<400> 41
cgttcttgat yaa 13

<210> 42
<211> 14
<212> DNA
<213> Artificial Sequence

<220>
<223> Probe based on primer NSR980/18 (ribosomal database project)

<400> 42
actttcgttc ttga 14

<210> 43
<211> 14
<212> DNA
<213> Artificial Sequence

<220>
<223> Primer based on primer NSR951/17 (ribosomal database project)

<400> 43
gyraatgctt tcgc 14

<210> 44
<211> 11
<212> DNA
<213> Artificial Sequence

<220>
<223> Probe based on primer NSR951/17 (ribosomal database project)

<400> 44
ttggyraatg c 11

<210> 45
<211> 14
<212> DNA
<213> Artificial Sequence

<220>
<223> Primer based on primer NSR581/18 (ribosomal database project)

<400> 45
ccgcggctgc tggc 14

<210> 46
<211> 9
<212> DNA
<213> Artificial Sequence

<220>
<223> Probe based on primer NSR581/18 (ribosomal database project)

<400> 46
attaccgcg 9

<210> 47
<211> 14
<212> DNA
<213> Artificial Sequence

<220>
<223> Primer based on primer NSR399/19 (ribosomal database project)

<400> 47
ggctccytct ccgg 14

<210> 48
<211> 12
<212> DNA
<213> Artificial Sequence

<220>
<223> Probe based on primer NSR399/19 (ribosomal database project)

<400> 48
tctcaggctc cy 12

<210> 49
<211> 20
<212> DNA
<213> Artificial Sequence

<220>
<223> Primer based on primers NSR399/19 and NSR387/18 (ribosomal database project)

<400> 49
cytctccggr rtcgarccct 20

<210> 50
<211> 18
<212> DNA
<213> Artificial Sequence

<220>
<223> Probe based on primers NSR399/19 and NSR387/18 (ribosomal database project)

<400> 50
tctcaggctc cytctccg 18

<210> 51
<211> 22
<212> DNA
<213> Artificial Sequence

<220>
<223> Yersinia enterocolitica yadA gene primer

<400> 51
ttctttcttt aattgcgcga ca 22

<210> 52
<211> 18
<212> DNA

<213> Artificial Sequence

<220>
<223> Yersinia enterocolitica yadA gene probe

<400> 52
tttttcaat ttctttct 18

<210> 53
<211> 21
<212> DNA
<213> Artificial Sequence

<220>
<223> Yersinia enterocolitica, vir F gene primer

<400> 53
actcatctta ccattaagaa g 21

<210> 54
<211> 23
<212> DNA
<213> Artificial Sequence

<220>
<223> Yersinia enterocolitica, vir F gene probe

<400> 54
aatatcaaca atadcatct tac 23

<210> 55
<211> 17
<212> DNA
<213> Artificial Sequence

<220>
<223> Yersenia enterocolitica ail gene primer

<400> 55
ccagtaatcc ataaagg 17

<210> 56
<211> 16
<212> DNA
<213> Artificial Sequence

<220>
<223> Yersenia enterocolitica ail gene probe

<400> 56
gcagcmccca gtaatc 16

<210> 57
<211> 20
<212> DNA
<213> Artificial Sequence

<220>

<223> Staphylococcus aureus nuc gene primer

<400> 57
tagccaagcc ttgacgaact 20

<210> 58
<211> 20
<212> DNA
<213> Artificial Sequence

<220>
<223> Staphylococcus aureus nuc gene probe

<400> 58
taagcaactt tagccaagcc 20

<210> 59
<211> 19
<212> DNA
<213> Artificial Sequence

<220>
<223> Salmonella sp. InvA primer

<400> 59
rctggtactg atcgataat 19

<210> 60
<211> 14
<212> DNA
<213> Artificial Sequence

<220>
<223> Salmonella sp. InvA probe

<400> 60
agacgrctgg tact 14

<210> 61
<211> 23
<212> DNA
<213> Artificial Sequence

<220>
<223> B. anthracis chromosomal gene rpoB primer

<400> 61
aacgatagct cctacatttg gag 23

<210> 62
<211> 19
<212> DNA
<213> Artificial Sequence

<220>
<223> B. anthracis chromosomal gene rpoB probe

<400> 62

gggggacaaa cgatagctc 19

<210> 63
<211> 17
<212> DNA
<213> Artificial Sequence

<220>
<223> Listeria monocytogenes mpl-gene primer

<400> 63
gcaacttccg gctcagc 17

<210> 64
<211> 20
<212> DNA
<213> Artificial Sequence

<220>
<223> Listeria monocytogenes mpl-gene probe

<400> 64
aatccaactc gcaacttccg 20

<210> 65
<211> 16
<212> DNA
<213> Artificial Sequence

<220>
<223> Listeria monocytogenes mpl-gene primer

<400> 65
gcatatccca wagttt 16

<210> 66
<211> 15
<212> DNA
<213> Artificial Sequence

<220>
<223> Listeria monocytogenes mpl-gene probe

<400> 66
tataagcata tccca 15

<210> 67
<211> 24
<212> DNA
<213> Artificial Sequence

<220>
<223> Listeria monocytogenes Hly-a 1primer

<400> 67
tagttctaca tcacctgaga caga 24

<210> 68

<211> 20
<212> DNA
<213> Artificial Sequence

<220>
<223> Listeria monocytogenes Hly-a 1probe

<400> 68
tgatatttgt tagttctaca 20

<210> 69
<211> 24
<212> DNA
<213> Artificial Sequence

<220>
<223> Listeria monocytogenes Hly-a 2 primer

<400> 69
ttccgaattc gcttttacga gagc 24

<210> 70
<211> 18
<212> DNA
<213> Artificial Sequence

<220>
<223> Listeria monocytogenes Hly-a 2 probe

<400> 70
ttttctacta attccgaa 18

<210> 71
<211> 22
<212> DNA
<213> Artificial Sequence

<220>
<223> Listeria monocytogenes Hly-a 3 primer

<400> 71
cttcttcttg cattttccct tc 22

<210> 72
<211> 20
<212> DNA
<213> Artificial Sequence

<220>
<223> Listeria monocytogenes Hly-a 3 probe

<400> 72
aaactaatga cttcttcttg 20

<210> 73
<211> 20
<212> DNA
<213> Artificial Sequence

<220>
<223> Shigatoxin mRNA primer

<400> 73
cacagactgc gtcagtgagg 20

<210> 74
<211> 18
<212> DNA
<213> Artificial Sequence

<220>
<223> Shigatoxin mRNA probe

<400> 74
cgctcttgcc acagactg 18

<210> 75
<211> 21
<212> DNA
<213> Artificial Sequence

<220>
<223> Shigatoxin mRNA primer

<400> 75
cgctgcagct gtattacttt c 21

<210> 76
<211> 10
<212> DNA
<213> Artificial Sequence

<220>
<223> Shigatoxin mRNA probe

<400> 76
gaaacgctgc 10

<210> 77
<211> 20
<212> DNA
<213> Artificial Sequence

<220>
<223> Shigatoxin mRNA primer

<400> 77
taaactgcac ttcagcaaat 20

<210> 78
<211> 18
<212> DNA
<213> Artificial Sequence

<220>
<223> Shigatoxin mRNA probe

<400> 78
agtcattatt aaactgca 18

<210> 79
<211> 20
<212> DNA
<213> Artificial Sequence

<220>
<223> Beta-lactamase mRNA 1 primer

<400> 79
tgcttaatca gtgaggcacc 20

<210> 80
<211> 18
<212> DNA
<213> Artificial Sequence

<220>
<223> Beta-lactamase mRNA 1 probe

<400> 80
ttaccaatgc ttaatcag 18

<210> 81
<211> 16
<212> DNA
<213> Artificial Sequence

<220>
<223> Beta-lactamase mRNA 2 primer

<400> 81
rtaaatgctt caataa 16

<210> 82
<211> 15
<212> DNA
<213> Artificial Sequence

<220>
<223> Beta-lactamase mRNA 2 probe

<400> 82
aaccctgrta aatgc 15

<210> 83
<211> 15
<212> DNA
<213> Artificial Sequence

<220>
<223> Primer based on primer BLR1686/20 (ribosomal database project)

<400> 83
cattttgccg agttc 15

<210> 84
<211> 15
<212> DNA
<213> Artificial Sequence

<220>
<223> Probe based on primer BLR1686/20 (ribosomal database project)

<400> 84
ggggccattt tgccg 15

<210> 85
<211> 15
<212> DNA
<213> Artificial Sequence

<220>
<223> Primer based on primer BLR1929/20 (ribosomal database project)

<400> 85
gaccgttata gttac 15

<210> 86
<211> 11
<212> DNA
<213> Artificial Sequence

<220>
<223> Probe based on primer BLR1929/20 (ribosomal database project)

<400> 86
cttaggaccg t 11

<210> 87
<211> 15
<212> DNA
<213> Artificial Sequence

<220>
<223> Primer based on primer BLR2595/20 (ribosomal database project)

<400> 87
ttctgaaccc agctc 15

<210> 88
<211> 12
<212> DNA
<213> Artificial Sequence

<220>
<223> Probe based on primer BLR2595/20 (ribosomal database project)

<400> 88
cgacgttctg aa 12

<210> 89
<211> 15
<212> DNA

<213> Artificial Sequence

<220>
<223> Primer based on primer BLR463/20 (ribosomal database project)

<400> 89
ctggttcact atcgg 15

<210> 90
<211> 11
<212> DNA
<213> Artificial Sequence

<220>
<223> Probe based on primer BLR463/20 (ribosomal database project)

<400> 90
cggtactggt t 11

<210> 91
<211> 16
<212> DNA
<213> Artificial Sequence

<220>
<223> Primer based on primer BLR2763/20 (ribosomal database project)

<400> 91
cttagatgct ttcagc 16

<210> 92
<211> 12
<212> DNA
<213> Artificial Sequence

<220>
<223> Probe based on primer BLR2763/20 (ribosomal database project)

<400> 92
cccgcttaga tg 12

<210> 93
<211> 12
<212> DNA
<213> Artificial Sequence

<220>
<223> Primer based on primer NLR677/16 (ribosomal database project)

<400> 93
gtccaccaag at 12

<210> 94
<211> 11
<212> DNA
<213> Artificial Sequence

<220>

<223> Probe based on primer NLR677/16 (ribosomal database project)

<400> 94
actcgtccac c 11

<210> 95
<211> 15
<212> DNA
<213> Artificial Sequence

<220>
<223> Primer based on primer NLR816/21(ribosomal database project)

<400> 95
tccgtgtttc aagac 15

<210> 96
<211> 13
<212> DNA
<213> Artificial Sequence

<220>
<223> Probe based on primer NLR816/21(ribosomal database project)

<400> 96
ccttggtccg tgt 13

<210> 97
<211> 17
<212> DNA
<213> Artificial Sequence

<220>
<223> Primer based on primer NLR818/25 (ribosomal database project)

<400> 97
tccgtgtttc aagacgg 17

<210> 98
<211> 15
<212> DNA
<213> Artificial Sequence

< 220 >
<223> Probe based on primer NLR818/25 (ribosomal database project)

<400> 98
ctccttggtc cgtgt 15

<210> 99
<211> 16
<212> DNA
<213> Artificial Sequence

<220>
<223> Primer based on primer NLR1126/22 (ribosomal database project)

<400> 99

ctgagggaaa cttcgg 16

<210> 100
<211> 12
<212> DNA
<213> Artificial Sequence

<220>
<223> Probe based on primer NLR1126/22 (ribosomal database project)

<400> 100
gctatcctga gg 12

<210> 101
<211> 16
<212> DNA
<213> Artificial Sequence

<220>
<223> Primer based on primer NLR1432/23 (ribosomal database project)

<400> 101
cacactcctt agcgga 16

<210> 102
<211> 15
<212> DNA
<213> Artificial Sequence

<220>
<223> Probe based on primer NLR1432/23 (ribosomal database project)

<400> 102
gttgttacac actcc 15

<210> 103
<211> 12
<212> DNA
<213> Artificial Sequence

<220>
<223> Primer based on primer NLR1694/17 (ribosomal database project)

<400> 103
ggaycgactn ac 12

<210> 104
<211> 11
<212> DNA
<213> Artificial Sequence

<220>
<223> Probe based on primer NLR1694/17 (ribosomal database project)

<400> 104
tctyaggayc g 11

<210> 105

<211> 13
<212> DNA
<213> Artificial Sequence

<220>
<223> Primer based on primer NLR2016/18 (ribosomal database project)

<400> 105
agacctgmtg cgg 13

<210> 106
<211> 12
<212> DNA
<213> Artificial Sequence

<220>
<223> Probe based on primer NLR2016/18 (ribosomal database project)

<400> 106
cttggagacc tg 12

<210> 107
<211> 19
<212> DNA
<213> Artificial Sequence

<220>
<223> Primer based on primer NLR2098/24 (ribosomal database project)

<400> 107
atccttwtcc cgaagttac 19

<210> 108
<211> 15
<212> DNA
<213> Artificial Sequence

<220>
<223> Probe based on primer NLR2098/24 (ribosomal database project)

<400> 108
agccaatcct twtcc 15

<210> 109
<211> 14
<212> DNA
<213> Artificial Sequence

<220>
<223> Primer based on primer NLR2362/20 (ribosomal database project)

<400> 109
agagcactgg gcag 14

<210> 110
<211> 12
<212> DNA
<213> Artificial Sequence

<220>
<223> Probe based on primer NLR2362/20 (ribosomal database project)

<400> 110
acattcagag ca 12

<210> 111
<211> 20
<212> DNA
<213> Artificial Sequence

<220>
<223> Primer based on primer NLR2368/26 (ribosomal database project)

<400> 111
agagcactgg gcagaaatca 20

<210> 112
<211> 13
<212> DNA
<213> Artificial Sequence

<220>
<223> Probe based on primer NLR2368/26 (ribosomal database project)

<400> 112
acattcagag cac 13

<210> 113
<211> 15
<212> DNA
<213> Artificial Sequence

<220>
<223> Primer based on primer NLR2571/21 (ribosomal database project)

<400> 113
cagggtcttc tttcc 15

<210> 114
<211> 11
<212> DNA
<213> Artificial Sequence

<220>
<223> Probe based on primer NLR2571/21 (ribosomal database project)

<400> 114
ctcaacaggg t 11

<210> 115
<211> 14
<212> DNA
<213> Artificial Sequence

<220>
<223> Primer based on primer NLR2781/19 (ribosomal database project)

<400> 115
ccagycaaac tccc 14

<210> 116
<211> 11
<212> DNA
<213> Artificial Sequence

<220>
<223> Probe based on primer NLR2781/19 (ribosomal database project)

<400> 116
ccgccccagy c 11

<210> 117
<211> 20
<212> DNA
<213> Artificial Sequence

<220>
<223> Primer based on primer NLR3033/24 (ribosomal database project)

<400> 117
acatcgaagr atcaaaaagc 20

<210> 118
<211> 10
<212> DNA
<213> Artificial Sequence

<220>
<223> Probe based on primer NLR3033/24 (ribosomal database project)

<400> 118
gccgacatcg 10

<210> 119
<211> 16
<212> DNA
<213> Artificial Sequence

<220>
<223> Primer based on primer NLR3113/24 (ribosomal database project)

<400> 119
ccagctcacg ttccct 16

<210> 120
<211> 15
<212> DNA
<213> Artificial Sequence

<220>
<223> Probe based on primer NLR3113/24 (ribosomal database project)

<400> 120
gtctaaaccc agctc 15

<210> 121
<211> 14
<212> DNA
<213> Artificial Sequence

<220>
<223> Primer based on primer NLR3284/21 (ribosomal database project)

<400> 121
ttagaggcgt tcag 14

<210> 122
<211> 14
<212> DNA
<213> Artificial Sequence

<220>
<223> Probe based on primer NLR3284/21 (ribosomal database project)

<400> 122
ttctgactta gagg 14

<210> 123
<211> 27
<212> DNA
<213> Artificial Sequence

<220>
<223> Primer for HIV RNA detektion (or + strand DNA detection)

<400> 123
tgctatgtca cttccccttg gttctct 27

<210> 124
<211> 20
<212> DNA
<213> Artificial Sequence

<220>
<223> Probe for HIV RNA detektion (or + strand DNA detection)

<400> 124
tagtagttcc tgctatgtca 20

<210> 125
<211> 28
<212> DNA
<213> Artificial Sequence

<220>
<223> Primer for HIV RNA detektion (or + strand DNA detection)

<400> 125
tactagtagt tcctgctatg tcacttcc 28

<210> 126
<211> 16
<212> DNA

<213> Artificial Sequence

<220>
<223> Probe for HIV RNA detektion (or + strand DNA detection)

<400> 126
tcctgaaggg tactag 16

<210> 127
< 211 > 20
<212> DNA
<213> Artificial Sequence

<220>
<223> Primer for Hepatitis B RNA (or + strand DNA detection)

<400> 127
cgaaccactg aacaaatggc 20

<210> 128
<211> 17
<212> DNA
<213> Artificial Sequence

<220>
<223> Probe for Hepatitis B RNA (or + strand DNA detection)

<400> 128
agccctacga accactg 17

<210> 129
<211> 23
<212> DNA
<213> Artificial Sequence

<220>
<223> Primer for Hepatitis B - strand DNA detection

<400> 129
tcaccatatt cttgggaaca aga 23

<210> 130
<211> 15
<212> DNA
<213> Artificial Sequence

<220>
<223> Probe for Hepatitis B - strand DNA detection

<400> 130
gcgggtcacc atatt 15

<210> 131
<211> 24
<212> DNA
<213> Artificial Sequence

<220>

<223> Primer for hepatitis C RNA + strand detection

<400> 131
ctcgcaagca ccctatcagg cagt 24

<210> 132
<211> 17
<212> DNA
<213> Artificial Sequence

<220>
<223> Probe for hepatitis C RNA + strand detection

<400> 132
cggggcactc gcaagca 17

<210> 133
<211> 24
<212> DNA
<213> Artificial Sequence

<220>
<223> Primer for Hepatitis C RNA - strand detection

<400> 133
gcagaaagcg tctagccatg gcgt 24

<210> 134
<211> 16
<212> DNA
<213> Artificial Sequence

<220>
<223> Probe for Hepatitis C RNA - strand detection

<400> 134
ctgtcttcac gcagaa 16

<210> 135
<211> 21
<212> DNA
<213> Artificial Sequence

<220>
<223> Primer for Hepatitis A RNA - strand detection

<400> 135
ttctgcagat tggcttacta c 21

<210> 136
<211> 18
<212> DNA
<213> Artificial Sequence

<220>
<223> Probe for Hepatitis A RNA - strand detection

<400> 136

ttcttgattc attctgca 18

<210> 137
<211> 19
<212> DNA
<213> Artificial Sequence

<220>
<223> Primer for hepatitis A RNA + strand detection

<400> 137
catgcaactc caaatctgt 19

<210> 138
<211> 17
<212> DNA
<213> Artificial Sequence

<220>
<223> Probe for hepatitis A RNA + strand detection

<400> 138
gagttaatcc atgcaac 17

<210> 139
<211> 21
<212> DNA
<213> Artificial Sequence

<220>
<223> Priemr for Rotavirus RNA + strand detection

<400> 139
cgaacaattc taatctaaga t 21

<210> 140
<211> 18
<212> DNA
<213> Artificial Sequence

<220>
<223> Probe for Rotavirus RNA + strand detection

<400> 140
ggtcacatcg aacaattc 18

<210> 141
<211> 23
<212> DNA
<213> Artificial Sequence

<220>
<223> Primer for Rotavirus - strand detection

<400> 141
atgtaccgtg aaagtgtgtc cgc 23

<210> 142

<211> 17
<212> DNA
<213> Artificial Sequence

<220>
<223> Probe for Rotavirus - strand detection

<400> 142
gatcatcatg taccgtg 17

<210> 143
<211> 15
<212> DNA
<213> Artificial Sequence

<220>
<223> Primer for Zaire Ebola virus RNA - strand

<400> 143
atgtggtgga ttata 15

<210> 144
<211> 24
<212> DNA
<213> Artificial Sequence

<220>
<223> Probe for Zaire Ebola virus RNA - strand

<400> 144
attataataa tcactgacat gcat 24

<210> 145
<211> 18
<212> DNA
<213> Artificial Sequence

<220>
<223> Primer for Zaire Ebola virus RNA + strand

<400> 145
atcggaactt ttctttct 18

<210> 146
<211> 18
<212> DNA
<213> Artificial Sequence

<220>
<223> Probe for Zaire Ebola virus RNA + strand

<400> 146
ttctttctca ttgaaaga 18

<210> 147
<211> 16
<212> DNA
<213> Artificial Sequence

<220>
<223> Primer for SARS virus RNA + strand

<400> 147
agagccttgt tcttgg 16

<210> 148
<211> 24
<212> DNA
<213> Artificial Sequence

<220>
<223> Probe for SARS virus RNA + strand

<400> 148
gttcttggtg tcaacgagaa aaca 24

<210> 149
<211> 14
<212> DNA
<213> Artificial Sequence

<220>
<223> Primer for SARS virus RNA - strand

<400> 149
cacgagtgag ttca 14

<210> 150
<211> 17
<212> DNA
<213> Artificial Sequence

<220>
<223> Probe for SARS virus RNA - strand

<400> 150
gagttcacgg agtgcac 17

<210> 151
<211> 18
<212> DNA
<213> Artificial Sequence

<220>
<223> gfpmb1-Fx

<400> 151
aattgtgagc ggataaca 18

<210> 152
<211> 27
<212> DNA
<213> Artificial Sequence

<220>
<223> gfpmb2-R

<400> 152
aagcttattt gtatagttca tccatgc 27

<210> 153
<211> 20
<212> DNA
<213> Artificial Sequence

<220>
<223> PRBA338F

<400> 153
actcctacgg gaggcagcag 20

<210> 154
<211> 12
<212> DNA
<213> Artificial Sequence

<220>
<223> 511R2

<400> 154
gctggcacgg ag 12

<210> 155
<211> 18
<212> DNA
<213> Artificial Sequence

<220>
<223> InvAF2

<400> 155
acagtgctcg tttacgac 18

<210> 156
<211> 18
<212> DNA
<213> Artificial Sequence

<220>
<223> InvAR1

<400> 156
actggtactg atcgataa 18

<210> 157
<211> 34
<212> DNA
<213> Artificial Sequence

<220>
<223> DNAB1
Biotin

<400> 157
gataacaatg atactagatt caattgtgag cgga 34

<210> 158
<211> 18
<212> DNA
<213> Artificial Sequence

<220>
<223> PNAB1

<400> 158
actagattcg attgtgag 18

<210> 159
<211> 11
<212> DNA
<213> Artificial Sequence

<220>
<223> PNAB2-reverse

<400> 159
gcggctgctg g 11

<210> 160
<211> 14
<212> DNA
<213> Artificial Sequence

<220>
<223> PNAInvA- reverse-1

<400> 160
agacgactgg tact 14

<210> 161
<211> 14
<212> DNA
<213> Artificial Sequence

<220>
<223> PNAInvA- reverse-1

<400> 161
agacgcctgg tact 14

## Description

### FIELD OF INVENTION

The present invention relates to a method for detecting the presence within a given environment of one or more target nucleic acid sequences while preventing or reducing interference from related nucleic acid species. Being based on the use of blocking probes the method will be of use for instance in the detection of gene expression in general, and in particular in the detection of microbial and viral activity and as a diagnostic tool. Furthermore the invention relates to compositions and kits of parts comprising components to be used in the method.

### GENERAL BACKGROUND AND PRIOR ART

PCR and related technologies have become widely used for the detection of nucleic acid molecules present in biological systems. In particular, the RT-PCR technology has proven to be a powerful tool when analysing activity characterised by transcription of specific genes, such as microbial activity and responses to environmental challenges (4, 5, 6) as well as the initiation and the progression of pathological processes. However, the specific nucleic acid molecules, which one aim at detecting, are often present at a very low amount in samples from biological systems and their detection is often complicated by the presence of molecular species having a structure related to that of the molecule to be detected. Such related molecular species may, even at a low amount, cause the generation of false results, either positive or negative. This clearly indicates the need for further development of sensitive, specific and efficient procedures.

For instance, when analysing low abundance RNA by RT-PCR procedures the elimination of contaminating DNA is of fundamental importance. Extraction of highly pure DNA-free RNA from biological samples usually presents a trade off between sample quality and sample quantity and co-extracted DNA is often present in excess of recovered RNA (7). Removal of residual contaminating DNA is commonly achieved by treating samples with DNase. However, DNase treatment is not well suited in a number of applications. When the starting material is an environmental sample, for instance, humic acids and other organic compounds present in such samples have been shown to inhibit DNases, while RNases are not inhibited (8), and the time involved in the DNase treatment could prove critical especially when dealing with highly labile mRNA. The ubiquitous presence of RNases and the nature of the RNA molecule, renders it highly susceptible to degradation and half-lives of these molecules typically range between seconds and a few minutes. Furthermore, some DNases possess residual RNase activity and when inactivating the DNase enzyme by heating to 70°C in the presence of $Mg^{2+}$, a chemical degradation of RNA is induced (9).

In order to overcome these problems numerous techniques have been developed which, in general, have focused on eliminating the amplification of molecular species structurally related to the target molecule. As for RT-PCR, a common approach is to use primers directed at the poly-A-tail which is added to most eukaryotic mRNA thereby selectively targeting the mRNA and not the corresponding genomic DNA. While this approach is widely used it can not, however, be applied to all biological systems. Thus, unlike eukaryotic mRNA, a poly-A tail is not added to the 3' end of procaryotic mRNAs rendering this technique inapplicable to, for instance, detection of bacterial activity. Detection of bacterial activity is the object of numerous diagnostic procedures as well as procedures for evaluating the quality of materials such as foodstuffs.

An alternative method is based on the quantitation of RNA transcripts using genomic DNA as an amplification competitor (23).

Increased specificity of PCR and RT-PCR reactions has been obtained by the use of nucleotide probes, which selectively block the amplification of molecular species having a sequence related to that of the target nucleotide sequence. In general, nucleic analogues such as PNA have been preferred when designing such blocking probes. PNA bind tightly to complementary sequences of DNA and the thermal stability of PNA/DNA duplexes at physiological ionic strength is generally higher (per base pair) than that of the corresponding DNA/DNA duplex (10, 11). This unique property and the inability of PNA to function as a primer for DNA polymerase allows PNA to play the role of a potent sequence specific PCR blocking molecule. Probes consisting of PNA have previously been used successfully to eliminate PCR product formation in a sequence-specific manner (10, 12, 13, 14). However, all probes previously used in attempts to block specific PCR reactions have been placed within the sequence of interest, their use again being restricted to applications such as blocking the amplification of undesired sequences containing nucleotide polymorphism(s) or intronic sequences not present in the target sequence. In conclusion there is still an explicit need for an efficient technique for preventing the amplification of unwanted nucleic acid species, a technique which can be applied universally.

### SUMMARY OF THE INVENTION

In the broadest sense, the present invention relates to a method for detecting the presence or absence of at least one target nucleic acid sequence in a sample that further contains a nucleic acid molecule comprising a sequence corresponding to the target nucleic acid sequence, the method comprising the step of contacting the sample with at least one nucleic acid probe that is capable of selectively binding to a continuum of at least a part of the nucleic acid molecule corresponding to the target nucleic acid sequence and a part of the nucleic acid molecule adjacent to said corresponding sequence.

In another aspect, the invention relates to a composition useful in the detection of the presence or absence of at least one target nucleic acid sequence in a sample that contains a nucleic acid molecule comprising a sequence corresponding to the target nucleic acid sequence, said composition comprising at least one nucleic acid probe that is capable of selectively binding to a continuum of at least a part of the nucleic acid molecule corresponding to the target nucleic acid sequence and a part of the nucleic acid molecule adjacent to said corresponding sequence.

In a further aspect, the invention pertains to a kit for detecting the presence or absence of at least one target nucleic acid sequence in a sample that contains a nucleic acid molecule comprising a sequence corresponding to the target nucleic acid sequence, said kit comprising at least one nucleic acid probe that is capable of selectively binding to a continuum of at least a part of the nucleic acid molecule corresponding to the target nucleic acid sequence and a part of the nucleic acid molecule adjacent to said corresponding sequence.

DEFINITIONS

As used herein, the term 'binding' refers to the association of molecules by intermolecular forces. In the present context binding of one nucleic acid to another refers to the interaction of two or more nucleic acids by base pairing, such as the formation of Watson-Crick base pairs or by alternative forms of base pairing such as the formation of Hoogsteen base pairs.

In the present context the term 'conserved sequence' refers to a base sequence in a nucleic acid molecule or an amino acid sequence in a protein that has remained relatively or essentially unchanged throughout evolution. The term may refer specifically to sequences, which are identical in one or more living species, such as one or more species within the same genus, family, order, subclass, class, subphylum, phylum, subkingdom, or kingdom.

The terms 'nucleic acid', 'nucleic acid molecule' and 'nucleic acid sequence' as used herein refer to an oligomer or polymer of ribonucleic acid (RNA) or deoxyribonucleic acid (DNA) or mimics/mimetics thereof. This term includes molecules composed of naturally-occurring nucleobases, sugars and covalent internucleoside (backbone) phosphodiester bond linkages as well as molecules having non-naturally occurring nucleobases, sugars and covalent internucleoside (backbone) linkages which function similarly or combinations thereof. Such modified or substituted nucleic acids are often preferred over native forms because of desirable properties such as, for example, enhanced cellular uptake, enhanced affinity for nucleic acid target and increased stability in the presence of nucleases and other enzymes, and are in the present context described by the terms "nucleic acid analogues" or "nucleic acid mimics". Preferred examples of nucleic acid mimics/mimetics are peptide nucleic acid (PNA-), Locked Nucleic Acid (LNA-), xylo-LNA-, phosphorothioate- , 2'-methoxy-, 2'-methoxyethoxy-, morpholino- and phosphoramidate- containing molecules or the like.

The nucleic acid, nucleic acid molecule or nucleic acid sequence may, for instance, be composed entirely of deoxyribonucleotides, entirely of ribonucleotides, entirely of nucleic acid mimics or analogues or chimeric mixtures thereof. The monomers are typically linked by internucleotide phosphodiester bond linkages. Nucleic acids typically range in size from a few monomeric units, e.g., 5-40, when they are commonly referred to as oligonucleotides, to several thousands of monomeric units. Whenever a nucleic acid or a nucleic acid sequence is represented, it will be understood that the nucleotides are in 5' to 3' order from left to right and that "A" denotes deoxyadenosine, "C" denotes deoxycytidine, "G" denotes deoxyguanosine, and "T" denotes thymidine, unless otherwise noted.

In general, as well as in the present context, the term 'target nucleic acid sequence' refers to a nucleic acid sequence that is the subject of hybridisation with a complementary polynucleotide, e.g. a primer or probe. As used herein 'target nucleic acid sequence' may also mean a nucleic acid sequence or a part of a nucleic acid sequence the presence of which is to be detected. Furthermore, a 'target nucleic acid sequence' may be a nucleic acid sequence or part of a nucleic acid sequence, which is complementary to a nucleic acid sequence the presence of which is to be detected. The sequence can be composed of DNA, DNA digests, RNA, and analogues thereof including sequences of synthetic nucleic acids and combinations of the aforementioned. More specifically, the target nucleic acid sequence may be a nucleic acid sequence which has been generated by transcriptioin and/or amplifcation *in vitro*; examples including complementary DNA (cDNA) and the first strand generated in strand specific PCR.

In general, as well as in the present context, the term 'molecule comprising a sequence corresponding to the target nucleic acid sequence' refers to any nucleic acid sequence that is not the target sequence but comprises a sequence resembling the target sequence. Examples of such sequences could be a nucleic acid fragment containing the target sequence or a sequence resembling the target sequence within its overall sequence as well as at least one further nucleotide.

In many embodiments of the present invention it is contemplated that the target nucleic acid sequence is a single stranded nucleic acid molecule for which a complementary strand can be generated. The target nucleic acid molecule may, however, also be a double stranded nucleic acid molecule.

In the present context, 'probe' means a nucleic acid or nucleic acid analogue which is useful for annealing or binding to a nucleic acid molecule, which is related to a target nucleic acid molecule such as a nucleic acid molecule corresponding to the target nucleic acid sequence or part hereof.

As used herein, the term 'annealing' refers to the association of single stranded nucleotides to form a double stranded structure such that a nucleotide in one strand of the double stranded structure undergoes specific Watson-Crick base pairing with a nucleotide on the opposite strand. The term also comprehends the pairing of nucleoside analogues, such as deoxyinosine, nucleosides with 2-aminopurine bases, and the like, that may be incorporated into the primers and probes of the invention.

The term 'upstream sequence' refers to a nucleic acid sequence, which is located at a position, which is 5' to a particular point or region within a nucleic acid molecule. In particular the term 'upstream' refers to sequences located in the opposite direction of the transcription reaction.

In the present context 'complementary sequence' or 'complement' refers to nucleotide sequences which will anneal to a nucleic acid molecule of the invention under stringent conditions. The term "stringent conditions" refers to general conditions of high, weak or low stringency. The term "stringency" is well known in the art and is used in reference to the conditions (temperature, ionic strength and the presence of other compounds such as organic solvents) under which nucleic acid hybridisations are conducted. With "high stringency" conditions, nucleic acid base pairing will occur only between nucleic acid fragments that have a high frequency of complementary base sequences, as compared to conditions of "weak" or "low" stringency.

As an example, high stringency hybridisation conditions comprise (1) low ionic strength and high temperature for washing, such as 0.015 M NaCl/0.0015 M sodium citrate, pH 7.0 (0.1×SSC) with 0.1% sodium dodecyl sulfate (SDS) at 50°C; (2) hybridisation in 50% (vol/vol) formamide with 5 x Denhardt's solution (0.1% (wt/vol) highly purified bovine serum albumin/0.1% (wt/vol) Ficoll/0.1% (wt/vol) polyvinylpyrrolidone), 50 mM sodium phosphate buffer at pH 6.5 and 5 x SSC at 42°C; or (3) hybridisation in 50% formamide, 5 x SSC, 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C with washes at 42°C in 0.2 x SSC and 0.1% SDS.

In the present context and when referring to nucleic acids or nucleic acid sequences derived from other nucleic acids or nucleic acid sequences, the term "derived" specifies that the nucleic acid or nucleic acid sequences are generated, wholely or in part, by polymerisation in vivo or in vitro of nucleosides using said other nucleic acid or nucleic acid sequence or part hereof as a template. Non-limiting examples of derived nucleic acids and derived nucleic acid sequences are sequences generated by DNA replication, transcription or reverse transcription, such as member of any of the functional classes of DNA and RNA sequences defined in the following detailed description of the invention. In particular, the terms "derived nucleic acid" and "derived nucleic acid sequence" encompass ribosomal RNA (rRNA) and messenger RNA (mRNA) sequences as well as sequences of complementary DNA (cDNA) and DNA sequences generated by in vitro polymerisation.

When referring to a nucleic acid molecules 'corresponding' to for instance the target nucleic acid sequence it is meant that this nucleic acid molecule or at least a part thereof is identical or has a certain degree of sequence identity to the target nucleic acid molecule or part thereof. The nucleic acid molecule or part thereof may thus be from 1-100%, such as at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, at least 99% or 100% identical to, for instance, the target nucleic acid molecule or part thereof.

When referring to 'identical sequences' herein, again it is meant sequences having a certain degree of sequence identity. The sequences may thus be from 1-100%, such as at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, at least 99% or 100% identical.

The term 'sequence identity' indicates a quantitative measure of the degree of homology between two amino acid sequences or between two nucleic acid sequences of equal length. If the two sequences to be compared are not of equal length they must be aligned to give the best possible fit, allowing the insertion of gaps or, alternatively, truncation at the ends of the polypeptide sequences or nucleotide sequences. The sequence identity can be calculated as $\frac{(N_{ref}-N_{dif})100}{N_{ref}}$, wherein $N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8). A gap is counted as non-identity of the specific residue(s), i.e. the DNA sequence AGTGTC will have a sequence identity of 75% with the DNA sequence AGTCAGTC

($N_{dif}$=2 and $N_{ref}$=8).

In all polypeptide or amino acid based embodiments of the invention the percentage of sequence identity between one or more sequences is based on alignment of the respective sequences as performed by clustalW software (http:/www.ebi.ac.uk/clustalW/index.html) using the default settings of the program. With respect to the nucleotide-based embodiments of the invention, the percentage of sequence identity between one or more sequences is also based on alignments using the clustalW software with default settings. For nucleotide sequence alignments these settings are: Alignment=3Dfull, Gap Open 10.00, Gap Ext. 0.20, Gap separation Dist. 4, DNA weight matrix: identity (IUB).

In the present context, by 'amplification reaction' is meant a reaction that produces one or more copies of a sequence of nucleic acids by repeated extension of a probe or primer.

'Extension' may occur by virtue of polymerisation of individual nucleotide monomers, as in PCR, or it may occur by the addition of prefabricated oligonucleotide segments, as in LCR, or by a combination of these as in gap LCR or Repair Chain Reaction (RCR). Though not essential to the invention, ideally the extension reactions are performed repeatedly and the extension products themselves may serve as templates to produce an exponential generation of products.

'$T_m$' can be defined as the temperature at which 50% of a nucleic acid and its perfect complement are in duplex. The denaturation of double stranded nucleic acids causes a shift in the absorbance of UV light at 260 nm wavelength, an effect which can be assayed by determining the optical density at 260 nm ($OD_{260}$). $T_m$ is defined as the temperature corresponding to 50% denaturation, i.e. where the ($OD_{260}$) is midway between the value expected for double stranded nucleic acids and the value expected for single stranded nucleic acids. The $T_m$ of perfectly complementary duplexes can be calculated as follows:

DNA:

$$T_m = 81.5 + 16.6(\log_{10}[Na^+]) + 0.41(\%GC) - 500/length$$

RNA; RNA-DNA:

$$T_m = 79.8 + 18.5(\log_{10}[Na^+]) + 0.58(\%GC) + 11.8(\%GC)^2 - 820/length$$

Oligonucleotides:

$$T_m = 2(\text{no. of AT pairs}) + 4(\text{no. of GC pairs})$$

As used herein, 'nucleic acid analogue' is understood to mean a structural analogue of DNA or RNA, designed to hybridise to complementary nucleic acid sequences (1). Through modification of the internucleotide linkage(s), the sugar, and/or the nucleobase, nucleic acid analogues may attain any or all of the following desired properties: 1) optimised hybridisation specificity or affinity, 2) nuclease resistance, 3) chemical stability, 4) solubility, 5) membrane-permeability, and 6) ease or low costs of synthesis and purification. Examples of nucleic acid analogues include, but are not limited to, peptide nucleic acids (PNA), locked nucleic acids "LNA", 2'-O-methyl nucleic acids, 2'-fluoro nucleic acids, phosphorothioates, and metal phosphonates. Nucleic acid analogues are described in (2) and (3).

As used herein, the term 'mRNA' means the presently known mRNA transcript(s) of a gene, and any further transcripts, which may be identified.

As used herein, the term 'gene' means the gene including exons, introns, non-coding 5'- and 3'-regions and regulatory elements and all currently known variants thereof and any further variants, which may be elucidated.

## DETAILED DESCRIPTION OF THE INVENTION

The object of the present invention is to provide an efficient method for detecting the presence within a given environment of one or more target nucleic acid sequences while preventing or reducing interference from related nucleic acid species such as the species defined herein. The method is based on the use of probes, which are capable of binding to the related nucleic acid species thereby preventing their detection.

Thus, in a broad sense the invention relates in a first aspect to a method for detecting the presence or absence of at least one target nucleic acid sequence in a sample that further contains a nucleic acid molecule which is related to the target nucleic acid molecule. More specifically this related nucleic acid molecule may comprise a sequence corresponding to the target nucleic acid sequence. The method comprises the step of contacting the sample with at least one nucleic acid probe that is capable of selectively binding to a continuum of at least a part of the nucleic acid molecule corresponding to the target nucleic acid sequence and a part of the nucleic acid molecule adjacent to said corresponding sequence. It is to be understood that the method according to the invention may be applied not only when the main objective is to detect the presence of a target nucleic acid but also for any purpose involving a step of detecting the presence or determining the amount of one or more given nucleic acids. It is further to be understood that 'a sequence corresponding to the target nucleic acid molecule' may be identical or complementary to the target nucleic acid sequence or a part hereof.

Inherent in the method of detecting the presence or absence of target nucleic acid sequences may be one or more steps of increasing the amount of target nucleic acids until reaching levels at which detection becomes possible. A crucial feature of the method may therefore be the presence of one or more extendable oligonucleotide primers. Whether occurring naturally as in a purified restriction digest or produced synthetically such primers should be able to act as a point of initiation of synthesis when placed under conditions in which a primer extension product complementary to a target sequence is induced. Therefore the method may further comprise contacting the sample with a first extendable primer.

According to this aspect of the invention annealing of at least one nucleic acid probe to the molecule comprising a sequence corresponding to the target nucleic acid sequence will prevent annealing of the extendable primer hereto and/or extension of the primer. Under the same conditions, however, the blocking probe will not affect attachment or annealing of the extendable primer to the target nucleic acid sequence. The blocking probe will thereby prevent PCR amplification of the molecule comprising a sequence corresponding to the target nucleic acid, without affecting amplification of the target nucleic acid itself.

It is envisaged that the target nucleic acid molecules have one or two defined ends and that the related nucleic acid molecules continue beyond these points. Therefore, the sequence-specific blocking probes can be designed, targeting regions of the related nucleic acid molecules immediately upstream of and proceeding through the sequences corresponding to a defined end of the target nucleic acid sequences. In RT-PCR, the one or two ends of the target nucleotide will correspond to either the RNA 5' end at the transcription initiation site or a RNA 5' end generated by RNA processing and one generated by the primer used in the RT-procedure. The corresponding chromosomal DNA sequence, on the other hand, will extend beyond the points corresponding to the RNA termini. Accordingly, in one embodiment of the invention the continuum of at least a part of the nucleic acid molecule corresponding to the target nucleic acid sequence and a part of the nucleic acid molecule adjacent to said corresponding sequence comprises a transcription initiation site and its upstream sequence.

Alternatively, the continuum of at least a part of the nucleic acid molecule corresponds to the target nucleic acid sequence and a part of the nucleic acid molecule adjacent to said corresponding sequence comprises a site containing the sequence targeted by an extendable primer and its upstream sequence. This may be the case for instance in strand specific-PCR, where one end of the target nucleotide will correspond to the end generated by the primer used in the first amplification cycle in a PCR-procedure. The corresponding complementary nucleic acid sequence, on the other hand, will extend beyond this point and act as target for blocking probes added prior to subsequent amplification cycles.

According to the main object of the invention hybridisation of the nucleic acid probe to the nucleic acid molecule corresponding to the target nucleic acid sequence reduces, hinders or prevents annealing of the at least one extendable primer hereto. This results in a reaction in which detection and/or amplification of the target nucleic acid sequence will prevail over detection and/or amplification of the nucleic acid molecule corresponding to the target nucleic acid sequence. Therefore, it is preferred that the first extendable primer and a part of the nucleic acid probe are both complementary to a 3' region within said part of the nucleic acid molecule corresponding to the target nucleic acid sequence. By a 3' prime region is meant a region in proximity of the 3' end of the given nucleic acid sequence

While the extendable primer and a part of the nucleic acid probe must be complementary to a suitable sequence within the target nucleic acid molecule this is to be interpreted so as to mean that the primer and probe must be sufficiently complementary to ensure annealing to their target sequences under stringent conditions. It is conceivable, however, that one may gain advantages by using mismatched or degenerate primers. One advantage could be, for instance, in a PCR reaction, the possibility of altering the melting temperature (Tm) of the (undesired) duplex between the blocking probe and the amplified target sequence by introducing mismatches in its sequence. Additionally the target sequence may contain degenerate positions within the primer target sequence requiring the use of degenerate primers for amplification. In particular, mismatched or degenerate primers may be used to target different, but closely related nucleic acid sequences, such as mRNAs from several closely related sources. Furthermore, such mismatches can be introduced in order to reduce the likelihood of the primer forming undesired secondary structures. It may therefore be preferred that the extendable primer has at least one mismatch relative to its complementary sequence in the target nucleic acid sequence. Alternatively, divergence may be generated by incorporating a tag sequence into the extendable primer. Since the tag should not prevent extension of the primer it is preferably located away from the 5' end of the primer, more preferably it is a 3' tag sequence. Addition of a tag sequence may further facilitate the use of higher primer annealing temperatures during the primer annealing step in PCR reactions, and eventually performing the probe annealing step and primer annealing step in a amplification reaction according to the present invention at more even temperatures, or even at the same temperature. The length of the mismatch or the 3' tag sequence may be from 1 - 20 nucleotides, such as 1, 2, 3, 4, 5, 6, 7, 8, 9, 10, 11, 12, 13, 14, 15, 16, 17, 18, 19 or 20 nucleic acids.

As mentioned, the nucleic acid probe must meet the dual criteria of being able to bind to the nucleic acid molecule corresponding to the target nucleic acid sequence under the desired conditions while not being able to bind to the target nucleic acid sequence. Therefore, the probe may be composed of two distinct parts, a first part, which is complementary to a region within the nucleic acid molecule corresponding to the target nucleic acid sequence, and a second part, which is complementary to a sequence of the nucleic acid molecule. Preferably, the first part of the probe is complementary to a 3' region within said part of the nucleic acid molecule corresponding to the target nucleic acid sequence, and a second part, which is complementary to a neighbouring sequence of the nucleic acid molecule.

When preparing nucleic acid probes and the extendable primers to be used in the present invention one will aim at designing probes and primers having favourable Tm-values. While, in general, longer probes and primers have a higher Tm they also hybridise with lower specificity at a given temperature as compared to shorter probes and primers. Therefore, the optimal length of the primers and probes is determined, at least in part, by the nucleotide sequence at which they are directed and may therefore vary within and between the different embodiment of the invention. Accordingly, the probes and primers of the invention may be more than or equal to any length between 3 and 200 nucleotides, such as more than or equal to 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200 nucleotides in length.

In some preferred embodiments of the invention, the length of the nucleic acid probes is from 11 to 35 nucleotides in length, whereas the first extendable primers are from 11 to 27 nucleotides in length. In other preferred embodiments the length of the nucleic acid probe is from 8 to 20 nucleotides in length, whereas the first extendable primer is from 11 to 27 nucleotides in length.

As mentioned, the nucleotide probe may have two distinct parts, a first part, which is complementary to a region within the nucleic acid molecule corresponding to the target nucleic acid sequence and a second part, which is complementary to a sequence of the nucleic acid molecule. Considerations similar to the above apply when determining the desired length of these parts. While the first part of the probe may constitute any proportion of the total length of the probe, the first part of the nucleic acid probe and the first extendable primer may be of equal length. This means that the first part of the nucleic acid probe and the first extendable primer are directed at the same 3' region within the target nucleic acid.

Ideally, in order to prevent annealing of the nucleic acid probe to the target nucleic acid, the hybrid of the target nucleic acid and the extendable primer should have a higher Tm- value than the putative hybrid between the nucleic acid probe and the target nucleic acid. It may be therefore be preferred that the first part of the nucleic acid probe is shorter than the first extendable primer.

Also, in a preferred embodiment of the invention, the $T_m$-value for the expected hybrid of the nucleic acid probe and the related nucleic acid molecule is higher than the $T_m$-value for the expected hybrid of the first extendable primer and the target nucleic acid sequence. This is to assure that annealing of the nucleic acid probe to the related nucleic acid molecule will take place at a temperature at which annealing of the first extendable primer does not occur. Thereby, annealing of the nucleic acid probe to the related nucleic acid sequence is favoured over annealing of the first extendable primer to its complementary sequence in the related nucleic acid molecule. Preferably the $T_m$ value for the expected hybrid of the nucleic acid probe and the related nucleic acid molecule is from 1 to 20°C, such as from 2 to 15°C, from 5 to 12°C or from 8 to 10°C higher than higher than the $T_m$- value for the expected hybrid of the first extendable primer and the target nucleic acid sequence.

Other approaches of assuring that the nucleic acid probe anneals to the related nucleic acid molecule prior to annealing of the extendable primers include the inclusion of two annealing steps. This will allow annealing of the nucleic acid probe and annealing of the first extendable primer to occur in two successive steps. In a preferred embodiment the step in which annealing of the nucleic acid probe occurs precedes the step in which annealing of the first extendable primer occurs. In order to obtain this sequential annealing a pre-primer annealing step may be introduced at a temperature, which is sufficiently high to only allow annealing of the probe. A subsequent lowering of the temperature allows the primer to anneal. In order to further promote annealing of the nucleic acid probe prior to annealing of the first extendable primer it is preferred that the nucleic acid probe is present in molar excess as compared to the primer.

In addition, it may be preferred that the sample containing the target nucleic acid sequence is contacted with the nucleic acid probe prior to contacting it with any one of the other components to be added, including extendable primers, nucleotides, enzymes, and buffers. Also, the sample containing the target nucleic acid sequence may preferably be contacted with the nucleic acid probe prior to any reverse transcription of nucleic acid molecules present in the sample or subsequent to such reverse transcription but prior to any amplification of the transcribed nucleic acid species, However, these are just examples of methods, which can be employed in order, for instance, to favour annealing of the nucleic acid probe over annealing of the first extendable primer, and the present invention is not limited to these methods.

In theory the nucleic acid blocking probe used in the present invention may be any nucleic acid molecule that is not (naturally or modified such) extended by the action of the used DNA polymerase. Examples of such blocking probes are probes (some of which require a 5' modification) comprising or consisting of DNA, RNA, peptide nucleic acid (PNA-), Locked Nucleic Acid (LNA-), Intercalating nucleic acids (INA), xylo-LNA-, phosphorothioate-, 2'-methoxy-, 2'-methoxyethoxy-, morpholino- and phosphoramidate- containing molecules or the like.

In a preferred embodiment of the present invention the nucleic acid probe is a nucleic acid analogue. In addition, it is preferred that the nucleic acid probe is a peptide nucleic acid (PNA). Peptide nucleic acids are compounds that in certain respects are similar to oligonucleotides and their analogues and thus may mimic DNA and RNA. In PNA, the deoxyribose backbone of oligonucleotides has been replace by a pseudo-peptide backbone. Each subunit, or monomer, has a naturally occurring or non-naturally occurring nucleobase attached to this backbone. One such backbone is constructed of repeating units of N- (2-aminoethyl) glycine linked through amide bonds. PNA hybridises with complementary nucleic acids through Watson and Crick base pairing and helix formation. The Pseudo-peptide backbone provides superior hybridization properties, resistance to enzymatic degradation and access to a variety of chemical modifications.

PNA binds both DNA and RNA to form PNA/DNA or PNA/RNA duplexes, respectively. The resulting PNA/DNA or PNA/RNA duplexes are bound with greater affinity than corresponding DNA/DNA or DNA/RNA duplexes as determined by their Tm's. In addition to increased affinity, PNA has also been shown to bind to DNA with increased specificity.

It is further envisaged that in certain applications the nucleic acid probe is preferably a locked nucleic acid (LNA). Structurally similar to RNA, LNA monomers are bicyclic compounds, which bear a methylene linker that connects the nucleotide sugar ring's 2'- oxygen to its 4'-carbon. Like PNA, LNA polymers obey standard base-pairing rules while offering greater stability and binding affinity to target nucleotide sequences.

As stated above the nucleic acid probe has a first part, which is complementary to a 3' region within the target nucleic acid. This is intended to mean that the nucleic acid probe will be able to hybridise to a 3' region within the target nucleic acid under stringent conditions c.f. the definition given above. Again, this means that the nucleic acid probe does not need to be an exact match of the sequence at which it is directed and it may in certain instances be preferred that the nucleic acid probe has at least one mismatch, such as 1, 2, 3, 4, 5, 6, 7, 8, 9, 10, 15, 20 or more mismatch(es), in its first part relative to its complementary sequence in the target nucleic acid sequence. One advantage could be, for instance, in a PCR reaction, the possibility of altering the melting temperature (Tm) of the (undesired) duplex between the blocking probe and the amplified target sequence by introducing mismatches in its sequence. In addition such mismatches can be introduced in order to reduce the likelihood of the probe forming undesired secondary structures. Additionally the first and/or second part of the probe may contain on or more mismatched or degenerate positions relative to its target sequence. Furthermore, the advantage could be, for instance, in a PCR reaction, the possibility of targeting degenerate positions within the probe target sequence. Another advantage could be related to the altering of the secondary structure of the probe.

The target nucleic acid sequence, the presence of which is to be detected using the method of the invention, may be virtually any type of nucleic acid molecule. According to the main aspect of the invention, however, the target nucleic acid sequence is a nucleic acid for which one or more complementary or partly complementary sequences and/or one or more identical or partly identical sequences exist. In particular, the target nucleic acid sequence may be one of two complementary nucleic acid sequences. More specifically, the target nucleic acid sequence is one of either strand of a double stranded nucleic acid sequence or one of either strand of a potentially double stranded nucleic acid sequence.

In particular, the target nucleic acid sequence may be an RNA or cDNA sequence to which an identical and/or complementary nucleic acid sequence exists. Also, the target nucleic acid sequence may be an RNA or cDNA sequence to which an identical double stranded nucleic acid sequence exists.

Several applications of the method according to the present invention may be devised in which the target nucleic acid sequence is a DNA sequence. Several main classes of DNA exist, both when classified by amount and by function. The DNA sequence may thus be a unique sequence (single copy, low copy, nonrepetitive DNA) which is present as one or a very few copies per genome. This is typically the case for most genes, including introns, regulatory sequences and other DNA of unknown function. Alternatively, the DNA may be a moderately repetitive DNA, which generally defines sequences present 10 - 10,000 copies per genome and include generally dispersed repeats corresponding to highly conserved multigene families (functional genes and pseudogenes) and transposable elements. Finally the DNA may be a highly repetitive DNA, which is defined as sequences present 100,000 to 1,000,000 copies per genome and normally found as tandem repeats although some superabundant (dispersed) transposable elements also fall into this class.

When classified by function one class of genomic DNA is genic DNA comprising DNA, which is actually expressed. Genic DNA may further be classified as mDNA (protein encoding DNA), rDNA, tDNA, snDNA, etc. representing the different classes of gene product. A second class of DNA is regulatory DNA comprising DNA whose role is in the regulation of gene expression (e.g. promoters and enhancers) or the regulation of DNA function (e.g. origins of replication, matrix associated regions). Another class of DNA is intergenic DNA or spacer DNA which comprises introns and the DNA, which separates the genes form each other. Satellite DNA is highly repetitive DNA found near centromeres, telomeres and at other sites, wherein some satellite DNA may play a role in chromosome function. Selfish DNA, which forms another functional class of DNA, is DNA whose role appears to be to mediate its own replication and survival within the genome, e.g. some satellite DNA, and transposable elements. Finally, DNA may be so-called junk DNA having no assigned function.

According to a preferred embodiment of the invention, however the target nucleic acid sequence is an RNA sequence. As for DNA several classes of RNA exist as summarised in the following. Major classes of RNA are thus messenger RNA (mRNA), heterogeneous nuclear RNA (hnRNA), transfer RNA (tRNA) and ribosomal RNA (rRNA). Whereas some mRNA-like transcripts are untranslated mRNA it is in general transcribed from protein encoding genes and carries the message for translation. hnRNA refers to prespliced RNA, the unmodified transcripts of eukaryotic genes so called because of its great diversity of size compared to tRNA and mRNA.

Several minor classes of RNA exists such as telomerase RNA, guide RNA (gRNA), antisense RNA (mRNA-interfering complementary RNA, micRNA), ribozymes, initiator RNA (iRNA), small nuclear RNA (snRNA) or uridine-rich RNA (U-RNA), small nucleolar RNA (snoRNA), and small cytoplasmic RNA (scRNA). It is envisaged that any class of nucleic acid comprising but not limited to those mentioned may constitute a target nucleic acid to be detected in a particular application of the method of the invention.

In a further preferred embodiment of the present invention the target nucleic acid sequence is derived from an RNA. Comprised hereby are target nucleic acids, which are synthesised on the basis of a RNA as wells as RNAs, which have been modified in any ways, and RNAs which are associated with other molecular species. Primarily, though, the target nucleic acid is synthesised by reverse transcription of an RNA belonging to any class of RNA including those mentioned above either *in vitro* or *in vivo* in an intact biological system. Accordingly, in this preferred embodiment the target nucleic acid sequence may be a cDNA sequence, such as the sequence of either strand of a double stranded cDNA. In a double stranded cDNA a first strand is generated by reverse transcription of an RNA, whereas the second strand is formed as the complementary strand by a DNA polymerase driven reaction using the first strand as a template.

The primary sequence of ribosomal RNAs vary considerably between species and a method, which is currently used for determining the presence of various organisms, is based on the amplification and detection of various parts of the ribosomal RNA from the organisms. In a preferred embodiment of the invention, therefore, the target nucleic acid sequence is derived from a ribosomal RNA.

Ribosomes are generally composed of a small and a large subunit. In E. *coli*, for instance, the small 30S subunit contains, besides proteins, a 16S rRNA whereas the large 50S subunit contains the 23S and 5S rRNAs. Eucaryotic ribosomes are larger and the small 40S subunit contains an 18S rRNA whilst the large 60S subunit contains three RNAs of 28S, 5.8S and 5S. Archaen ribosomes resemble those of bacteria but some genera contain additional subunits similar to those of eukaryotes. In a currently preferred embodiment of the invention the target nucleic acid sequence is derived from 16S ribosomal RNA. It is envisaged that the method may employ the use of universal rRNA-directed primers and probes for the purpose of detecting the presence of any organism or it may use primers and probes which are specific for rRNA from a given species.

In another preferred embodiment of the present invention the target nucleic acid is derived from a messenger RNA. It will be understood that the method may be applied to the detection of abundant messengers as well as rare messengers for which it is particularly useful due to its high sensitivity. In particular, it may be preferred that the target nucleic acid is derived from a mRNA which does not possess a poly(A)-tail, such as a mRNA from a prokaryotic species.

In praticular applications of the method according to the invention, the target nucleic acid is identical to a nucleic acid sequence, which is specific for one or more microbial species, or its complementary nucleic acid sequence. It follows that the method can then be used in the detection and/or quantification of these one or more microbial species in a given environment.

In particular applications it may also be preferred that the target nucleic acid is identical to a nucleic acid sequence, which is conserved among several microbial species, or its complementary nucleic acid sequence.

In currently preferred embodiments of the invention use of the method is directed at determining the presence of a pathogen or a pathogenic species within a given environment. In these embodiments the microbial species may comprise one or more pathogenic species.

The above mentioned characteristics of the target nucleic acid sequence may alternatively apply to part of the sequence only, such as the part to which said first and/or said second extendable primer is capable of annealing. Alternatively expressed, the first and/or second extendable primer may be complementary to a nucleic acid sequence, which is specific for one or more microbial species, or identical to a sequence, which is complementary to said specific nucleic acid sequence. The first and/or second extendable primer may likewise be complementary to a nucleic acid sequence, which is conserved among several microbial species, or identical to a nucleic acid sequence complementary said conserved nucleic acid sequence

In a prominent embodiment of the present invention the target nucleic acid sequence is derived from a bacterial messenger RNA. Inherent in this embodiment is use of the method of the invention in order to evaluate the presence of bacteria in different environments, such as in different biological systems.
In a currently most preferred embodiment of the invention, the target nucleic acid sequence is identical to a nucleic acid sequence which is derived from the invA gene of *Salmonella Spp.* or identical to the complementary sequence of said derived sequence. In particular the target nucleic acid may be identical to a nucleic acid sequence derived from an mRNA of the invA gene, such as a corresponding cDNA, or to the complementary sequence of said derived sequence. The following sequences exemplifies probes and primers and primers useful for this application:

Probe: H-AGA-CGA/C-CTG-GTA-CT-$NH_2$
Forward primer: 5'ACA GTG CTC GTT TAC GAC-3'
Reverse primer: 5'-ACT GGT ACT GAT CGA TAA-3'

Other embodiments of the invention pertain to detection, quantification and/or characterisation of components of biotic processes, hereunder components involved in the degradation or conversion of certain molecular species. In these embodiments, the target nucleic acid may be identical to a nucleic acid sequence derived from a gene, the product of which is involved in the degradation or conversion of an undesired molecular species, or identical to the complementary sequence of said derived sequence. In particular the target nucleic acid may be identical to a nucleic acid sequence derived from an mRNA of said gene, such as a corresponding cDNA, or to the complementary sequence of said derived sequence. It is contemplated that the method according to the invention may be applied within the realm of bioremediation, that is in the prevention or reduction of environmental contaminations using for instance micro-organisms capable of degrading or converting the contaminants.

Accordingly, it may be preferred that said gene product is involved in the degradation or conversion of one or more pesticides. In a preferred embodiment of the invention illustrating this concept, the target sequence is identical to a nucleic acid sequence derived from the tfdA gene of *Ralstonia euthophus* JMP134 or a part of hereof, such as a corresponding cDNA, or identical to the complementary sequence of said derived sequence. The tdfA gene product is critical in the degradation of certain pesticides MCPA and 2,4-D. The following exemplifies primer and probe sequences which can be applied when analysing tfdA gene expression.
Probe: Ac-CGT-TCC-AGC-GAT-GC
Forward primer: 5'AC(GC)-GAG-TTC-TG(CT)-GA(CT)-ATG-3'
Reverse primer: 5'CAG-GAT-GCC-GGT-ACA-CGA- 3'

The tfdA extendable primers have been designed for the combined targeting of several tfdA genes. As an alternative to the above sequence, the reverse primer sequence may be: 5'AGC-GAT-GCC-GGT-ACA-CGA- 3' , representing, according to "The Entrez Nucleotides Data Base", a perfect match with the published tfdA sequence from *Ralstonia euthophus* JMP134. The use of degenerate primers or primers carrying a 3' tag sequence has been decribed in the preceding text as general embodiments of the invention.

For a number of other applications and combinations of target nucleic acid and nucleic acid molecule comprising a sequence corresponding to the target nucleic acid sequence particular sets of extendable primers and probes may be devised. Examples of such sets of primers and probes are listed in table 1. Primers are designed on the basis of sequences from the "The Entrez Nucleotides database" which is a collection of sequences from several sources, including GenBank, RefSeq, and PDB, as well as primers shown in the "European ribosomal RNA database" (referred to as ribosomal database project).

Table 1.

| **Specific bacterial 16S and 23S** | | |
|---|---|---|
| **Target nucleic acid** | **Primer** | **Probe** |
| *Salmonella* 16S | TATTAACCACAACACCT-3' | AATTGCTGCGGTTATTAA |
| *Yersenia sp.* 16S | CCGATGGCGTGAGGCCC | CTGGGCACATCCGAT |
| *Campylobacter sp.* 16S | CATTGTAGCACGTGTGTC | TTGTATATGCCATTGTAGCA |
| *Clostridium perfringens* 16S: | GGGTACCGTCATTATCTTCCC | GGCTTCCTCCTTGGGTAC |
| *Legionella sp.* 16S | CCAACAGYWAGTTGACATCG T | TTTCATATAACCAACAGYWA G |
| *Listeria monocytogenes* 16S: | ATAGTTTTATGGGATTAGC | ACTGAGAATAGTTTTAT |
| *Mycobacterium Sp.* 16S: | GCCCGTATCGCCCGCACGCT CACA | CTCTAGTCTGCCCGTATC |
| *Vibrio vulnificus* 23S | TACTTGTAACCCATC | AAGATACTTGTA |

| Universal bacterial 16S | | |
|---|---|---|
| **Target nucleic acid** | **Primer** | **Probe** |
| Universal Bacterial 16S | GCTGGCACGGAG | GCGGCTGCTGG |
| Based on primer BSR1541/20 (ribosomal database project) | TGATCCAGCCGCA | GAGGTGATCC |
| Based on primer BSR1407/16 (ribosomal database project) | GCGGTGTGTRC | GACGGGCGGT |
| Based on primer BSR1114/16 (ribosomal database project) | TGCGCTCGTTRC | GGGTTGCGC |
| Based on primer BSR798/15 (ribosomal database project) | GTATCTAATCCC | GGGGTATCTA |
| Based on primer BSR65/17 (ribosomal database project) | CTTGCATGTRTTA | TCGACTTGCATG |
| Based on primer BSR357/15 (ribosomal database project) | CTGCCTYCCGTA | CTGCTGCCT |
| Based on primer BSR534/18 (ribosomal database project) | CCGCGGCTGCTGGC | ATTACCGCG |

| Universal eukaryote nuclear 18S | | |
|---|---|---|
| **Target nucleic acid** | **Primer** | **Probe** |
| Based on primer NSR1787/18 (ribosomal database project) | GGTTCACCTACRG | CYGCAGGTTCAC |
| Based on primer NSR1642/16 (ribosomal database project) | GGCGGTGTGTRC | GACGGGCG |
| Based on primer NSR1147/20 (ribosomal database project) | CAATTYYTTTRAGTTT | CCGTCAATTYY |
| Based on primer NSR1438/20 (ribosomal database project) | ATCACAGACCTGTTAT | GGGCATCACA |
| Based on primer NSR980/18 (ribosomal database project) | CGTTCTTGATYAA | ACTTTCGTTCTTGA |
| Based on primer NSR951/17 (ribosomal database project) | GYRAATGCTTTCGC | TTGGYRAATGC |
| Based on primer NSR581/18 (ribosomal database project) | CCGCGGCTGCTGGC | ATTACCGCG |
| Based on primer NSR399/19 (ribosomal database project) | GGCTCCYTCTCCGG | TCTCAGGCTCCY |
| Based on primers NSR399/19 and NSR387/18 (ribosomal database project) | CYTCTCCGGRRTCGARCCCT | TCTCAGGCTCCYTCTCCG |

| Specific mRNA/cDNA | | |
|---|---|---|
| **Target nucleic acid** | **Primer** | **Probe** |
| *Yersinia enterocolitica yadA* gene | TTCTTTCTTTAATTGCGCGAC A | TTTTTTCAATTTCTTTCT |
| *Yersinia enterocolitica,* vir F gene | ACTCATCTTACCATTAAGAAG | AATATCAACAATACTCATCTT AC |
| *Yersenia enterocolitica ail* gene | CCAGTAATCCATAAAGG | GCAGCMCCCAGTAATC |
| *Staphylococcus aureus* nuc gene | TAGCCAAGCCTTGACGAACT | TAAGCAACTTTAGCCAAGCC |
| *Salmonella* sp. InvA | RCTGGTACTGATCGATAAT | AGACGRCTGGTACT |
| *B. anthracis* chromosomal gene *rpoB* | AACGATAGCTCCTACATTTGG AG | GGGGGACAAACGATAGCTC |

(continued)

| Specific mRNA/cDNA | | |
|---|---|---|
| **Target nucleic acid** | **Primer** | **Probe** |
| *Listeria monocytogenes* mpl-gene | GCAACTTCCGGCTCAGC | AATCCAACTCGCAACTTCCG |
| *Listeria monocytogenes* mpl-gene | GCATATCCCAWAGTTT | TATAAGCATATCCCA |
| *Listeria monocytogenes Hly-a* 1 | TAGTTCTACATCACCTGAGAC AGA | TGATATTTGTTAGTTCTACA |
| *Listeria monocytogenes Hly-a* 2 | TTCCGAATTCGCTTTTACGAG AGC | TTTTCTACTAATTCCGAA |
| *Listeria monocytogenes Hly-a 3* | CTTCTTCTTGCATTTTCCCTTC | AAACTAATGACTTCTTCTTG |
| Shigatoxin mRNA | CACAGACTGCGTCAGTGAGG | CGCTCTTGCCACAGACTG |
| | CGCTGCAGCTGTATTACTTTC | GAAACGCTGC |
| | TAAACTGCACTTCAGCAAAT | AGTCATTATTAAACTGCA |
| Beta-lactamase mRNA 1: | TGCTTAATCAGTGAGGCACC | TTACCAATGCTTAATCAG |
| Beta-lactamase mRNA 2 | RTAAATGCTTCAATAA | AACCCTGRTAAATGC |

| Universal bakterial 23S | | |
|---|---|---|
| **Target nucleic acid** | **Primer** | **Probe** |
| Based on primer BLR1686/20 (ribosomal database project) | CATTTTGCCGAGTTC | GGGGCCATTTTGCCG |
| Based on primer BLR1929/20 (ribosomal database project) | GACCGTTATAGTTAC | CTTAGGACCGT |
| Based on primer BLR2595/20 (ribosomal database project) | TTCTGAACCCAGCTC | CGACGTTCTGAA |
| Based on primer BLR463/20 (ribosomal database project) | CTGGTTCACTATCGG | CGGTACTGGTT |
| Based on primer BLR2763/20 (ribosomal database project) | CTTAGATGCTTTCAGC | CCCGCTTAGATG |

| Universal eukaryote nuclear 28S | | |
|---|---|---|
| **Target nucleic acid** | **Primer** | **Probe** |
| Based on primer NLR677/16 (ribosomal database project) | GTCCACCAAGAT | ACTCGTCCACC |
| Based on primer NLR816/21(ribosomal database project) | TCCGTGTTTCAAGAC | CCTTGGTCCGTGT |
| Based on primer NLR818/25 (ribosomal database project) | TCCGTGTTTCAAGACGG | CTCCTTGGTCCGTGT |
| Based on primer NLR1126/22 (ribosomal database project) | CTGAGGGAAACTTCGG | GCTATCCTGAGG |
| Based on primer NLR1432/23 (ribosomal database project) | CACACTCCTTAGCGGA | GTTGTTACACACTCC |
| Based on primer NLR1694/17 (ribosomal database project) | GGAYCGACTNAC | TCTYAGGAYCG |
| Based on primer NLR2016/18 (ribosomal database project) | AGACCTGMTGCGG | CTTGGAGACCTG |
| Based on primer NLR2098/24 (ribosomal database project) | ATCCTTWTCCCGAAGTTAC | AGCCAATCCTTWTCC |

(continued)

| Universal eukaryote nuclear 28S | | |
|---|---|---|
| **Target nucleic acid** | **Primer** | **Probe** |
| Based on primer NLR2362/20 (ribosomal database project) | AGAGCACTGGGCAG | ACATTCAGAGCA |
| Based on primer NLR2368/26 (ribosomal database project) | AGAGCACTGGGCAGAAATCA | ACATTCAGAGCAC |
| Based on primer NLR2571/21 (ribosomal database project) | CAGGGTCTTCTTTCC | CTCAACAGGGT |
| Based on primer NLR2781/19 (ribosomal database project) | CCAGYCAAACTCCC | CCGCCCCAGYC |
| Based on primer NLR3033/24 (ribosomal database project) | ACATCGAAGRATCAAAAAGC | GCCGACATCG |
| Based on primer NLR3113/24 (ribosomal database project) | CCAGCTCACGTTCCCT | GTCTAAACCCAGCTC |
| Based on primer NLR3284/21 (ribosomal database project) | TTAGAGGCGTTCAG | TTCTGACTTAGAGG |

| Virus detection (strand specific detection) | | |
|---|---|---|
| **Target nucleic acid** | **Primer** | **Probe** |
| HIV RNA detektion (or + strand DNA detection) | TGCTATGTCACTTCCCCTTGG TTCTCT | TAGTAGTTCCTGCTATGTCA |
| HIV RNA detektion (or + strand DNA detection) | TACTAGTAGTTCCTGCT-ATGT CACTTCC | TCCTGAAGGGTACTAG |
| Hepatitis B RNA (or + strand DNA detection) | CGAACCACTGAACAAATGGC | AGCCCTACGAACCACTG |
| Hepatitis B - strand DNA detection | TCACCATATTCTTGGGAACAA GA | GCGGGTCACCATATT |
| Hepatitis C RNA + strand detection | CTCGCAAGCACCCTATCAGG CAGT | CGGGGCACTCGCAAGCA |
| Hepatitis C RNA - strand detection | GCAGAAAGCGTCTAGCCATG GCGT | CTGTCTTCACGCAGAA |
| Hepatitis A RNA - strand detection | TTCTGCAGATTGGCTTACTAC | TTCTTGATTCATTCTGCA |
| Hepatitis A RNA + strand detection | CATGCAACTCCAAATCTGT | GAGTTAATCCATGCAAC |
| Rotavirus RNA + strand detection | CGAACAATTCTAATCTAAGAT | GGTCACATCGAACAATTC |
| Rotavirus - strand detection | ATGTACCGTGAAAGTGTGTCC GC | GATCATCATGTACCGTG |
| Zaire Ebola virus RNA - strand | ATGTGGTGGATTATA | ATTATAATAATCACTGACATG CAT |
| Zaire Ebola virus RNA + strand | ATCGGAACTTTTCTTTCT | TTCTTTCTCATTGAAAGA |
| SARS virus RNA + strand | AGAGCCTTGTTCTTGG | GTTCTTGGTGTCAACGAGAA AACA |
| SARS virus RNA - strand | CACGAGTGAGTTCA | GAGTTCACGGAGTGCAC |

In table 1, Y = C or T, S = C or G, M =C or A, R = A or G, W= A or T, K = G or T.

As is the case for the target nucleic acid sequence, the nucleic acid molecule comprising a sequence corresponding to the target nucleic acid sequence may be any type of nucleic acid molecule. In a preferred embodiment of the invention, however, the nucleic acid molecule comprising a sequence corresponding to the target nucleic acid sequence is a genomic DNA molecule c.f. the classification of genomic DNA presented above.

When binding to a nucleic acid molecule which in part corresponds to the target nucleic acid sequence the probe preferably prevents or reduces annealing of the forward extendable primer. The probe may, however, also be designed so as to prevent or reduce annealing of the reverse primer thereby abolishing the synthesis of a nucleic acid molecule, which is complementary in sequence to the target nucleic acid molecule. In this case the nucleic acid molecule comprising a sequence corresponding to the target nucleic acid sequence is a nucleic acid molecule comprising a sequence which is complementary to the target nucleic acid sequence.

As indicated above the method according to the present invention may be applied to conventional PCR reactions. A prerequisite for obtaining non-linear amplification of the target sequence is the use of a forward primer together with a reverse primer. Therefore the method may further comprise a step of contacting the sample with a second extendable primer which is complementary to a part of the complementary strand of said target nucleic acid sequence and detecting the target nucleic acid sequence subsequent to its amplification. In the present context, the forward primer thus serves as a primer for the synthesis of a nucleic acid sequence, which is complementary to target nucleic acid or a part hereof whereas the reverse primer is a primer for the synthesis of a nucleic acid molecule, which is identical to the target nucleic acid or a part hereof.

In a preferred embodiment of the invention the $T_m$-value for the expected hybrid of the second extendable primer and the complementary strand of said target nucleotide sequence is comparable to the $T_m$-value for the expected hybrid of the nucleic acid probe and the nucleic acid molecule comprising a sequence corresponding to the target nucleic acid sequence. This will make annealing of both the probe and the second extendable primer possible during the pre-primer annealing step serving the purpose of preventing re-annealing of the PCR products during the pre-primer annealing step. Furthermore it assures that replication of the nucleic acid molecule comprising a sequence corresponding to the target nucleic acid sequence will proceed past the sequence corresponding to the target nucleic acid and into an adjacent part of the molecule.

Amplification of the target nucleic acid typically occurs through cycles comprising the steps of increasing or lowering the temperature of the sample to levels which will allow denaturation of nucleic acid hybrids and double stranded nucleic acids, annealing of the primers and probes, and replication of the relevant nucleic acids. In the method according to the invention the target nucleotide sequence and its complementary strand are thus amplified in at least one thermocycle comprising the steps of primer annealing, primer extension and denaturation.

It is to be understood that the temperature at which each step of the thermocycle is conducted may have to be optimised depending on the amplification method, the nature of the target nucleic acids, the extendable primers and the nucleic acid probe. Typically, however, the temperature of the denaturation step, the pre-primer annealing step, the annealing step and the elongation step is within the range of 90 - 99°C, 50 - 70°C, 40 - 65°C and 70 - 75°C, respectively. The duration of each step is from 5 seconds to 15 minutes, from 5 seconds to 5 minutes, from 10 seconds to 10 minutes and from 15 seconds to 15 minutes, respectively.

Typically, blocking probes are added to a final concentration within the range of 0.1 to 20 $\mu$M whereas the primers are added to a final concentration within the range of 0.1 to 2 $\mu$M.

While the Polymerase Chain Reaction is the most widely applied method for *in vitro* enzymatic amplification of nucleic acids, it is not unique. Several other techniques have been developed for particular applications. Ligase Chain Reaction, for instance, may be used for the sensitive detection of a DNA sequence and for discrimination between alleles. Two primers are used which anneal at adjacent sites on target DNA. If the target sequence is present, the primers can be ligated together and will act as sites for further annealing and ligation. The strand displacement reaction is similar to the PCR reaction but does not require a denaturation step in each cycle. After extension, the primers are separated from their extension product by restriction endonuclease cleavage and further extension then proceeds by strand displacement from the resulting nick. Transcription-based amplification or nucleic acid sequence-based amplification (NASBA) is a rapid amplification procedure based on transcription and reverse transcription. The target nucleic acid is amplified by standard techniques using primers carrying a phage promoter and further amplification is then carried out by transcription using phage RNA polymerase. The transcripts are reverse transcribed to generate cDNA for further amplification. The amplification of nucleic acids which is associated with the method of the present invention may occurs by any *in vitro* amplification method, in particular in a PCR or a LCR procedure.

Numerous advances and extensions to basic PCR strategy have been developed. The PCR amplification of cDNA is termed RT-PCR as it involves the initial reverse transcription of RNA prior to amplification. Several applications of the RT-PCR technique have been devised such as the identification of differentially expressed genes known as differential display PCR or mRNA fingerprinting. Another PCR based approach is asymmetric PCR wherein one of the primers becomes depleted after a limited number of cycles. It is to be understood that the method of the present invention may be applied to virtually any PCR based technique including those mentioned. In a preferred embodiment of the invention, however, the amplification of a target nucleic acid sequence takes place as an integrated part of a RT-PCR procedure. When applying the method of the present invention to a RT-PCR procedure, the main objective may be to prevent amplification of genomic DNA that is identical or partly identical to the complementary DNA formed. Alternatively, the method may be applied in order to prevent or reduce the amplification of contaminating nucleic acid species, which unintentionally have been introduced into a sample during preparation or handling procedures.

In a further preferred embodiment of the invention the sample is contacted with the nucleic acid probe prior to reverse transcription of the RNA. In this case, which may be referred to as a so-called 'one-step' RT-PCR procedure, the nucleic acid probe will not be able to interfere with annealing of the extendable primers to the RNA sequence, which is subject to reverse transcription and subsequent amplification and detection. In the present context, the term 'one-step' indicates that generation of cDNA from the RNA and amplification of the cDNA *takes place in successive steps of a single procedure during* which there may be no need to add further reagents. The possibility of conducting the method of the present invention as a one-step RT-PCR procedure exists when the nucleic acid probe is designed to anneal to a sequence of the genomic DNA comprising the transcription initiation site of a gene and the first extendable primer has a sequence within the cDNA corresponding to the transcription initiation sequence as its target.

Optionally, detection of the presence of the target nucleic acid is performed using a real-time PCR system. All real-time PCR systems rely upon the detection and quantification of a fluorescent reporter, the signal of which increases in direct proportion to the amount of PCR product in a reaction. In a simple and economical format, that reporter is the double- strand DNA-specific dye SYBR® Green (Molecular Probes). SYBR Green binds doublestranded DNA, and upon excitation emits light. Thus, as a PCR product accumulates, fluorescence increases. The two most popular alternatives to SYBR Green are TaqMan® and molecular beacons, both of which are hybridisation probes relying on fluorescence resonance energy transfer (FRET) for quantification. TaqMan Probes are oligonucleotides that contain a fluorescent dye, typically on the 5' base, and a quenching dye, typically located on the 3' base. When irradiated, the excited fluorescent dye transfers energy to the nearby quenching dye molecule rather than fluorescing, resulting in a nonfluorescent substrate. TaqMan probes are designed to hybridise to an internal region of a PCR product. During PCR, when the polymerase replicates a template on which a TaqMan probe is bound, the 5' exonuclease activity of the polymerase cleaves the probe. This separates the fluorescent and quenching dyes and FRET no longer occurs. Fluorescence increases in each cycle, proportional to the rate of probe cleavage. Molecular beacons also contain fluorescent and quenching dyes, but FRET only occurs when the quenching dye is directly adjacent to the fluorescent dye. Molecular beacons are designed to adopt a hairpin structure while free in solution, bringing the fluorescent dye and quencher in close proximity. When a molecular beacon hybridises to a target, the fluorescent dye and quencher are separated, FRET does not occur, and the fluorescent dye emits light upon irradiation.

By recording the amount of fluorescence emission at each cycle, it is possible to monitor the PCR reaction during exponential phase where the first significant increase in the amount of PCR product correlates to the initial amount of target template. The higher the starting copy number of the nucleic acid target, the sooner a significant increase in fluorescence is observed. A significant increase in fluorescence above the baseline value measured during the 3-15 cycles (by default) indicates the detection of accumulated PCR product. A fixed fluorescence threshold is set significantly above the baseline, which can be altered by the operator. The parameter $C_T$ (threshold cycle) is defined as the cycle number at which the fluorescence emission exceeds the fixed threshold.

One additional possibility, which may be particularly feasible considering the advance of the real-time PCR technology, is thus to detect the presence of the target nucleic acid molecule in a quantitative or semi-quantitative manner. Typically, this is accomplished by determining the amount of product generated by amplification of the target nucleic acid sequence, or in real-time PCR the corresponding $C_T$-value,relative to the amount of product or the $C_T$-value obtained when amplifying a nucleic acid sequence, which is present in the sample in a known quantity. Thus, the method of the present invention may further comprise steps of determining the quantity of a target nucleotide sequence in a plurality of nucleic acid molecules.

The method of the present invention may advantageously be applied when seeking to detect the presence of microbial species in general and also when aiming at identifying or characterising a microbial species present. Moreover the technique is applicable when seeking to characterise nucleic acid sequences present within a known microbial species, or nucleic acids which are synthesised in response to the presence of a microbial species. It may therefore for certain applications be preferred that the first extendable primer is complementary to a nucleotide sequence, which is specific for one or more microbial species. By a microbial species is meant a micro-organism such as a bacterium, archean, protistan, virus as well as fungi or related small eucaryot species.

Of particular relevance may also be the detection or determination of nucleic acid sequences conferring resistance towards antibiotics whether such resistance is desired or not. Accordingly, the first extendable primer may be complementary to a nucleotide sequence of a gene the product of which confers resistance towards one or more antibiotics.

An objective of employing the method according to the present invention may for instance be to detect the presence of microbial infections possibly causing a poisoning reaction. Therefore, it may be preferred that the first extendable primer is complementary to a nucleotide sequence of a gene encoding a microbial toxin. A large array of microbial toxins is known including among others the bacterial toxins. The latter include the exotoxins which are conventionally classified into three types: enterotoxins, cytotoxins, and neurotoxins, as well as the endotoxins. A few examples of bacterial toxins are botulinium toxin, tetanus toxin, diphtheria toxin, streptolysosins O and S, Cholera toxin, heat-labile enterotoxin, heat-stable enterotoxin, and shiga toxin.

The method of the invention may be employed in the detection of nucleic acid molecules in samples, which are obtained from a multicellular organism and may be used as part of a diagnostic procedure. In particular, the sample may contain body fluids such as blood, serum, urine or saliva as well as body tissue, for example skin, muscle or bone, or it may contain extracts or homogenates of such body tissue. Preferably, the sample is obtained from a mammal, such as a primate. In particular, the sample may be obtained from a human.

Alternatively the method of the invention may be applied to samples, which are obtained from a food source or form a source of drinking water and the objective may be to evaluate these sources of food and/or drinking water. Of particular relevance would be the detection of contaminants within such environments, which may constitute a health issue, such as the detection of pathogenic organisms.

As for application of the method according to the invention to the detection of Salmonella Spp, salmonella live in the intestinal tracts of humans and other animals including birds, and are transmitted by the oral-fecal route, that is via intake of foods contaminated with animal feces. Contaminated foods are often of animal origin, such as beef, poultry, milk, or eggs, but all foods, including vegetables may become contaminated. For detection of salmonella, samples may thus be obtained from virtually any food sources, in particular any of those mentioned above.

As mentioned above the microbial species may be a bacterial species. The bacterial species may belong to any of the constituent groups of the archaebacteria or eubacteria. Constituent groups of archaebacteria are the Sulfolobus-Thermoproteus group, the methanogen-halophile group and the Thermoplasma. Constituent groups of eubacteria may be the gram-positive eubacteria, the purple bacteria - pseudomonad group, spirochetes, the bacterioides-cytophaga group, the cyanobacteria, the green sulphur bacteria, the green non-sulphur bacteria, the sulphur-reducers and muxobacteria, the radioresistant micrococci and the planctomyces group. More specifically, the bacterial species may be selected from the group comprising: *Salmonella spp., Escherichia spp., Listeria spp., Shigella spp., Legionella spp., Campylobacter spp., Staphylococcus spp., Clostridium spp., Streptococcus spp., Vibrio spp., Bacillus spp., Yersenia spp., Mycobacterium spp.*

Other than being a bacterial species the microbial species may also be an animal, plant or bacterial virus and may belong to any family within these classes. Furthermore the virus may be a DNA virus, such as a dsDNA virus or a ssDNA virus, as well as a RNA replicating virus, such as a dsRNA virus, a (-)ssRNA virus or a (+)ssRNA virus. Alternatively, the virus may be a RNA- or DNA-retroid virus. The method of the invention may be conveniently used in strand-specific RT-PCR procedures for instance for detection of retroviral RNA and/or detection of one of two complementary strands of a viral genome. In particular, a strand specific RT-PCR approach is useful when seeking to detect viruses, which utilise the synthesis of replicative RNA, a negative-strand RNA complementary to the genomic nucleic acid, as a template for virus replication.

Although the invention is exemplified in the present text by the use of only one nucleic acid probe it is to be understood that, at least in theory, any number of different probes may be used in the method of the present invention. In a PCR reaction, for instance, it may be feasible to use one probe which prevent annealing of a forward primer to a nucleic acid molecule corresponding to the target nucleic acid sequence in combination with another probe that prevents annealing of a reverse primer to a nucleic acid molecule corresponding to a sequence which is complementary to the target nucleic acid sequence. Thus, the method according to the invention may further comprise the step of contacting the sample with at least one second nucleic acid probe capable of binding to a continuum of at least a part of the nucleic acid molecule corresponding to a sequence complementary to the target nucleic acid sequence and a part of the nucleic acid molecule adjacent to said corresponding sequence.

As described above for the nucleic acid probe the at least one second nucleic acid probe may be composed of two parts. Therefore, in a preferred embodiment of the invention the at least one second nucleic acid probe comprises a first part, which is complementary to a 3' region within said part of the nucleic acid molecule corresponding to a sequence complementary to the target nucleic acid sequence and a second part, which is complementary to a neighbouring sequence of the nucleic acid molecule.

The use of more than one nucleic acid probe may also be instituted when more than one molecular species, which are structurally related to a target molecule are present especially in cases where the use of only one probe is insufficient. Finally, one can also imagine that several different target nucleic acids are to be detected in a multiplex reaction, one or more sets of primers and probes being used for each target nucleic acid.

A second aspect of the invention relates to a composition for use, or a composition useful in the detection of the presence or absence of at least one target nucleic acid sequence in a sample that contains a nucleic acid molecule comprising a sequence corresponding to the target nucleic acid sequence, said composition comprising at least one nucleic acid probe that is capable of selectively binding to a continuum of at least a part of the nucleic acid molecule corresponding to the target nucleic acid sequence and a part of the nucleic acid molecule adjacent to said corresponding sequence.

In addition to the nucleic acid probe, a composition according to the invention may comprise any number of additional components. In particular the composition may comprise at least one member selected from the group consisting of polymerases, nucleotides, buffers, salts, stabilising agents, diluents and agents for detection of nucleic acids. These may be reagents, which recognise specific sequences or reagents, which recognise nucleic acids in a non-specific manner. Examples of the former are oligonucleotides or nucleic acid analogues optionally bearing detectable labels. Examples of the latter are antibodies to nucleic acids.

In another aspect of the invention, a kit for detecting the presence or absence of one or more target nucleic acid sequence in a sample that contains one or more related nucleic acid molecules. Again, a related nucleic acid molecule may be a nucleic acid molecule comprising a sequence corresponding to the target nucleic acid sequence. The kit may contain one or more of the reagents necessary for detecting, hereunder identifying, the particular target nucleic acid or acids including primers, non-extendable blocking probes, reaction buffers, as well as directions giving a protocol for carrying out the procedure. In particular, the kit may comprise at least one nucleic acid probe that is capable of selectively binding to a continuum of at least a part of the nucleic acid molecule corresponding to the target nucleic acid sequence and a part of the nucleic acid molecule adjacent to said corresponding sequence.

The kit may further comprise at least one member selected from the group consisting of polymerases, nucleotides, buffers, salts, stabilising agents, diluents and agents for detection of nucleic acids. Again, these may be reagents, which recognise specific sequences or reagents, which recognise nucleic acids in a non-specific manner. Examples of the former are oligonucleotides or nucleic acid analogues optionally bearing detectable labels. Examples of the latter are antibodies to nucleic acids.

It is to be understood that any part of the description relating to the first aspect of the invention, namely the method itself, will also apply to the composition and the kit of parts described above.

FIGURE LEGENDS

**Figure 1.**
Inhibitor probe principle: Following annealing of the blocking probe to a nucleic acid molecule comprising a sequence corresponding to the target nucleic acid sequence (DNA) attachment of the primer to said molecule and/or extension of said primer is blocked under conditions where attachment and/or extension of said primer to the target nucleic acid sequence (cDNA) is possible. Thereby amplification of the target nucleic acid is allowed while amplification of sequence corresponding to the target nucleic acid sequence is blocked.

**Figure 2.**
RT - PCR using blocking probes: In the RT-reaction reverse transcription of an RNA template is performed using a first extendable primer is. In the subsequent PCR reactions the blocking probe will block the DNA strand containing the sequence corresponding to the target sequence and the amplification products of the complementary DNA strand. Thus, the PCR reaction results in exponential amplification of only the target sequence.

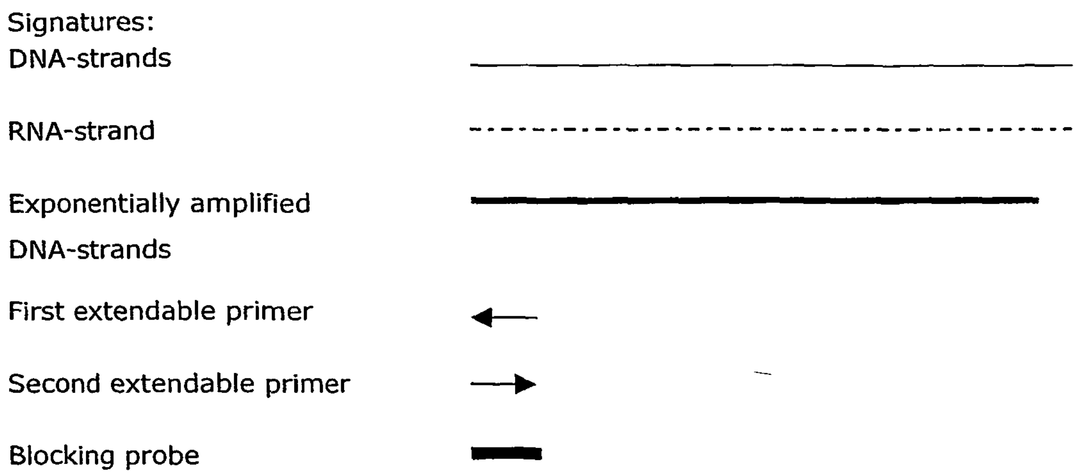

**Figur 3.**

Strand specific PCR using blocking probes: In the first PCR reaction amplification of the target strand is done using a first extendable primer. In the following PCR reactions the blocking probe will block the DNA strand containing the sequence complementary to the target sequence and the amplification products of this complementary DNA strand. Thus, the PCR reaction results in exponential amplification of only the target strand.

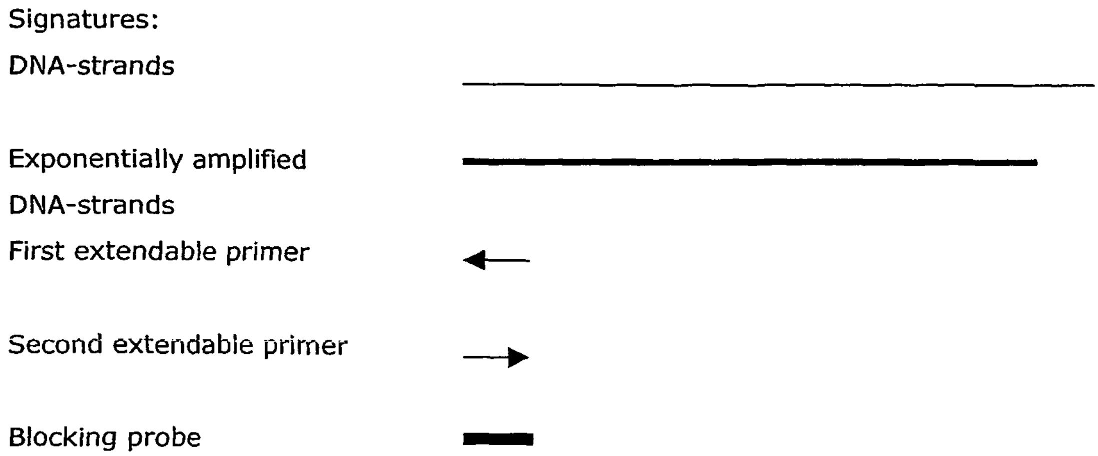


**Figure 4.**

Ethidium bromide stained PCR-products using mixtures of genomic *gfp* containing DNA derived from *P. putida* OUS82 UCB55 and *gfp* internal standard as template. Conc. of internal standard was kept constant in all tubes while the conc. of genomic *gfp* was consistently halved in tubes 1-8. **Upper panel:** No blocking probe added. Approx. equal band intensities measured in lane 7 implying equal amount of templates initially present in tube 7. **Middle panel:** PNAB1 added (8,0 μM final conc.) to PCR reactions. Approx. equal band intensities in lane 1 implying approx. equal amplification in the initial presence of more than $2^6$ times more *gfp* template than internal standard template. **Lower panel:** DNAB1 added (8,0 μM final conc.) to PCR reactions. Approx. equal intensities in lane 4 implying some suppression of the *gfp* template.

**Figure 5.**

Ethidium bromide stained RT-PCR product bands using DNase treated (lanes 1-4) and untreated (lanes 5-8) total RNA extracts from *P. putida* OUS82 UCB55. RT enzyme was excluded from reactions 3, 4, 7 and 8 (products shown in lanes 3, 4, 7, and 8 respectively) and PNAB1 (8.0 μM final conc.) was added to PCR-reactions 2, 4, 6 and 8 (products shown in lanes 2, 4, 6 and 8, respectively).

**Figure 6.**

Ethidium bromide stained RT-PCR product bands using as template DNase treated (lanes 6-10, 16-20), and untreated (lanes 1-5, and 11-15) total RNA extracts from 5 parallel cultures of E. *coli* SP308/pKJK10. Increasing levels of IPTG were used to induce each culture. PNAB1 was added (8.0 μM final conc.) to reactions 11-20 (lanes 11-20, respectively).

**Figure 7.**

Ethidium bromide stained RT-PCR product bands using as template DNase treated (lanes 1-4 a, b), and untreated (lanes 5-8 a, b) total RNA extracts from exponentially growing (a) and starving (b) cultures of E. *coli* SP308/pKJKIO. RT enzyme was excluded from reactions 3, 4, 7 and 8 (products shown by lanes 3, 4, 7, and 8 a, b respectively) and addition of PNAB2-reverse (2.0 μM final conc.) to PCR-reactions 2, 4, 6 and 8 was performed (products shown by lanes 2, 4, 6 and 8, a, b respectively).

**Figure 8.**

Agarose gel with bands representing products originating from Real-time PCR analysis presented in Tabel 3. Lane 1: Direct nucleic acid extract (RNA and DNA), with no reverse transcriptase treatment but with addition of PNAInvA-reverse 1. Lane 2: like lane 1 but no PNA addded *(corresponding to table 3, 2, row, No PNA);* Lane 3: Reverse transcribed after DNA'se treatmentwith PNA and lane 4: like 3 but with no PNA addition *(corresponding to table 3,*

*3. row, with PNA and no PNA, respectively);* Lane 5: Reverse transcribed, no DNA'se treatment, PCR with PNA probe and lane 6 like Lane 5 but without PNA addition *(corresponding to table 3, 4. row, with PNA and no PNA, respectively),* Lane 7 Molecular ladder; Lane 8: negative control.

EXAMPLES

**Materials and experimental conditions**

Strains:

Two bacterial strains, *Escherichia coli* SP308/pKJK10 (16) and *Pseudomonas putida* OUS82 UCB55 (16), harbouring the same *gfp* construct (17) were used in this study. The *gfp* gene in *E. coli* SP308/pKJK10 was under the control of the LacI repressible promoter $P_{A1/04/03}$ (18) and is inducible by the gratuitous inducer of the lac operon isopropyl-ß-D-thiogalactopyranoside (IPTG). In the *P. putida* strain the gfp-gene was constitutively transcribed. The S. *typhimorium* invA gene was constitutively transcribed.

DNA and RNA extractions:

Pure cultures of *P. putida* OUS82 wt (16, 17) and *P. putida* OUS82 UCB55 were grown overnight in 10 % LB broth and DNA was extracted using the Fast Soil DNA purification kit (Bio 101, Vista. Calif.) modified for liquid samples.

Total RNA was extracted using the FASTRNA blue kit (Bio 101) in accordance with the manufacturers instructions. *P. putida* OUS82 UCB55 was grown to exponential phase in 10% LB broth. Approx. $10^7$ cells (200 μl, $OD_{600}$ = 0.1) were sampled and total RNA was extracted.

Five parallel cultures of E. *coli* SP308 cells were grown exponentially in minimal glucose media supplemented with casaminoacids (0.5 % final conc.). DNA was extracted using the Fast Soil DNA purification kit (BI0101). At time 0, the five cultures received IPTG to final concentrations of 0 μM, 100 μM, 500 μM, 1000 μM or 2000 μM, respectively. Cell numbers and fluorescence per cell was monitored every 30 min. by flow cytometry (BD FACSCalibur, FSC= E01, SSC= 350, FL1= 566 ($\lambda$=530±15 nm). All on log gain). After 2 hours of exponential growth in the presence or absence of IPTG, duplicates of $10^7$ cells were sampled and immediately frozen in liquid nitrogen. Samples were kept at -80°C until total RNA was extracted.

Total RNA was further extracted from $10^7$ starved *E. coli* SP308 cells grown to lag phase in minimal glucose media supplemented with casaminoacids and thereafter incubated for 3 days.

Pure culture of S. *thyphimorium* was grown in full strength LB over night and the DNA was extracted using the MO BIO ultraclean soil DNA extraction kit (MO-BIO, Solane Beach, CA). All DNA extractions were done as recommended by the manufacturer except replacing the soil with pure culture. For RNA isolation S. *typhimorium* was grown overnight at RT in full strength LB medium and 300 μl culture was washed and resuspended in 300 μl phosphate buffer pH 7.4. RNA was extracted from the suspended cells using the BI0101 Fast RNA kit for soil in accordance with the manufacturers instruction.

All RNA samples were extracted to a final volume of 100μl. All RNA samples were reverse transcribed immediately after extraction or treated with DNase and thereafter reverse transcribed. All reagents used in the procedures was either treated and labelled RNase free by supplier or treated with 0.1% DEPC Diethyl pyrocarbonate (Sigma, St. Louis, MO, USA) prior to autoclaving to produce RNase free reagents.

DNase treatment of RNA samples from *P. putida* and E. *coli* was performed using the Amplification Grade DNase I kit (SIGMA, St. Lou., Miss.) in accordance with the manufacturers instructions. DNase treatment of samples from S. *typhimorium* was performed using RQ1 Rnase-free DNase (Promega, Madison, WI, USA).

Primers and blocking probes:

Primers and blocking probes used in this study are shown in table 2. *gfp*mb1-Fx was designed to target the leading strand sequence corresponding to the expected start sequence of the reverse transcribed *gfp* messenger -10 bases from the pribnow box. *gfp*mb2-R was placed approximately 770 bases downstream of *gfp*mb1-Fx. To prevent re-annealing of PCR products during the pre-primer-annealing step and to assure that replication of the genomic DNA lagging strand proceeds through the transcription initiation point, the reverse primer was designed to anneal in the pre-primer-annealing step of the PCR reaction.

Table 2:

| Primer/Probe | Sequence |
|---|---|
| gfpmb1-Fx | 5'AATTGTGAGCGGATAACA3' |
| gfpmb2-R | 5'AAGCTTATTTGTATAGTTCATCCAT GC 3' |
| PRBA338F | 5'ACTCCTACGGGAGGCAGCAG 3' |
| 511R2 | 5'GCTGGCACGGAG 3' |
| InvAF2 | 5'ACA GTG CTC GTT TAC GAC-3' |
| InvAR1 | 5'ACT GGT ACT GAT CGA TAA-3' |
| DNAB1 | 5'GATAACAATGATACTAGATTCAATT GTGAGCGGA 3'Biotin |
| PNAB1 | H-ACTAGATTCGATTGTG AG --$NH_2$ |
| PNAB2-reverse | H-GCGGCTGCTGG-$NH_2$ |
| PNAInvA-reverse-1 | H-AGA-CGA/C-CTG-GTA-CT-$NH_2$ |

Two different blocking probes DNAB1 and PNAB1 were designed targeting the leading strand region immediately upstream of and just overlapping the *gfpmb*1-Fx target. To avoid secondary structures detrimental to synthesis of the PNA probe a mismatch was introduced in the PNA probe. The mismatch was introduced to the region of the probe overlapping the *gfp* cDNA sequence. By this placement of the mismatch, the expected $T_m$ value of the PNA/cDNA hybrid (1 mismatch/10match) was lowered while the expected $T_m$ of the PNA/genomicDNA hybrid was not affected to the same extent (1 mismatch/18match). An A to G change was chosen as such a substitution was expected to have the least impact on $T_m$ (10).

The primers PRBA338F and 511R2 were used to amplify rRNA from E. *coli*. PNAB2-reverse was designed to block amplification of rDNA from the reverse primer. This approach limits the use of the system to a two step RT-PCR procedure, as a probe present in the RT step would inhibit cDNA synthesis. However the general applicability of the developed technique is greatly enhanced as it liberates the researcher from design restrictions arising due to the transcription initiation placement of the primer/probe pair when blocking the forward primer.

The primers invAF2 and invAR1a/invAR1b were designed to target the invA gene from Salmonella sp. involved in the invasiveness of Salmonella; this gene appears to be a valid target for most Salmonella (Chen and Griffith 2001; Malorney et *al.* 2003)

Primers and DNAB1 were custom primers of HPSF purity purchased from MWG Biotech AG, Germany. PNAB1 and PNAB2-reverse and PNAinvA-reverse-1 were purchased from Applied Biosystems.

PCR conditions:

*Gfp*-targeted PCR was performed in a final volume of 25 μl, containing 17.5 μl or, when blocking probes were added, 15.5 μl DNase/RNase-free water (Bio 101, Vista, Calif.), 2.5 μl 10X Ampli *Taq* PCR-buffer (Applied Biosystems, Norwalk, Conn.), 0.125 μl of each primer (in a 0.1 mM solution), 2.5 μl of DNase-free bovine serum albumin (Amersham Pharmacia, Uppsala, Sweden), 1 μl of Gene Amp 10 mM deoxynucleoside triphosphate mixture (Applied Biosystems), 0.125 μl of Ampli Taq polymerase (Applied Biosystems) and 1 μl of template DNA. Blocking probe was added to a final concentration of 8.0 μM (2 μl of a 0.1 mM solution). Cycling conditions; 10 min at 95°C; 35 cycles of 30s at 95°C, 30s at 63°C, 30s at 57°C and 1 min at 72°C; 6 min at 72°C and final cooling at 4°C. rRNA-targetted PCR cycling conditions; 10 min at 95°C; 35 cycles of 30s at 92°C, 30s at 58°C, 30s at 53°C and 1 min at 72°C; 6 min at 72°C and final cooling at 4°C.

PCR of cDNA was done as described above except for that 3 μl of the obtained cDNA product was used in a 50 μl reaction containing; 29.7 μl or, when blocking probe was added, 25.7 μl RNase-free water (Bio 101), 9.4 μl 5X RT-PCR buffer, 3.2 μl 25 mM magnesium chloride, 3.7 μl 10 mM dNTP blend, 0.25 μl of the primers (in a 0.1 mM solution), 0.5 μl Ampli Taq Gold DNA polymerase (2.5 units) and when appropriate 4.0 μl PNAGFP1 (in a 0.1 mM solution to achieve 8.0 μM final conc.).
PCR cycling conditions as above.

*Gfp*-targeted RT-PCR was performed as a two step reaction using the GeneAmp Gold RNA PCR reagent kit (Applied Biosystems) in accordance with the manufacturers instructions. 1 μl of template RNA was reverse transcribed in a 20 μl reaction volume (15 min. at 45°C) containing: 8.1 μl RNase-free water (Bio 101), 4.0 μl 5X RT-PCR buffer, 2.0 μl 25 mM magnesium chloride, 2.0 μl 10 mM dNTP blend, 0.5 μl RNase inhibitor (10 units), 2.0 μl 100 mM DTT, 0.1 μl gfpmb2-R primer and 0.3μl MultiScribe reverse transcriptase (15 units). 3 μl of the obtained product was used in the subsequent PCR step containing; 29.7 μl or, when blocking probe was added, 25.7 μl RNase-free water (Bio 101), 9.4 μl 5X RT-PCR buffer, 3.2 μl 25 mM magnesium chloride, 3.7 μl 10 mM dNTP blend, 0.25 μl of the primers (in a 0.1 mM solution), 0.5 μl Ampli Taq Gold DNA polymerase (2.5 units) and when appropriate 4.0 μl PNAB1 (in a 0.1 mM solution to achieve 8.0 μM final conc.). PCR cycling conditions as above.

rRNA-targeted RT-PCR was performed as a two-step procedure using ImProm-II reverse transcriptase and *Taq* DNA polymerase (Promega, Madison, WI.) in accordance with the manufacturers instructions. 1 μl of heat-denatured (70°C for 5 min.) template RNA was reverse transcribed in a 20 μl reaction volume (30 min. at 45°C) containing: 10.4 μl RNase-free water (Promega), 4.0 μl 5X Improm-II reaction buffer, 2.4 μl 25 mM magnesium chloride, 1.0 μl 10 mM dNTP blend, 0.2 μl (20 pmol) 511R-2 primer and 1.0 μl Improm-II reverse transcriptase. 2 μl of the obtained product was used in the subsequent PCR step containing; 22.75 μl or, when blocking probe was added, 21.75 μl Nuclease-free water (Promega), 25 μl 2X PCR Master mix (Promega), 0.125 μl of the primers (in a 0.1 mM solution) and when appropriate 1.0 μl PNAB2-reverse (in a 0.1 mM solution to achieve 2.0 μM final conc.). PCR cycling conditions as above.

For Real Time PCR of *S. thyphimorium* InvA cDNA PCR reactions in a total volume of 25 μl was prepared by combining: 13.25 μl $dH_2O$, 2,5 μl 10X reaction buffer (BIO RAD); 0.75 μl $MgCl_2$, 1 μl 5mM dNTP mixture , 0.5 μl 20 pmol/μl InvAF2 and InvAR1a/ InvAR1b primers; 0.25 μl 1/1000 diluted cybergreen and 0.25 μl iTaq polymerase (BIO-RAD). PCR conditions in the BIORAD real time termocycler: 10 min at 95°C; 35 cycles of 30s at 95°C, 30s at 67°C, 30s at 55°C and 30s min at 72°C; followed by a melting program with 0.5°C increase between each measurement using FAM490 filters, finally followed by cooling at 4°C PNAB1 and PNAB2-reverse stock solutions were vortexed and heated to 50°C for 10 min. prior to addition to PCR mastermix. Estimates of PCR-product abundance were obtained by quantification of ethidium bromide-stained PCR product band intensities on 1.5% agarose gels using an UV-visible-light recording camera and the Multi-Analyst software (Bio-rad, Hercules, Calif.)

**Example 1**

**PNAB1 inhibits the formation of genomic derived *gfp* PCR product.**

PCR results when using template DNA from *P. putida* OUS82 wt and *P. putida* OUS82 UCB55 confirmed the *gfp* specificity of the primers (data not shown).

Formation of *gfp* PCR product using *P. putida* OUS82 UCB55 genomic DNA as template was monitored at annealing step temperatures between 55°-65°. Most product was observed at annealing temperatures 57° and 58°, while slightly less product was observed at 59°, and no product was observed at 60° (data not shown). The PCR cycling program (see PCR conditions) was decided on the basis of these results. The probes require the sequence upstream of the forward-primer target sequence in order to block amplification. Once a *gfp* PCR-product of genomic DNA origin is produced it is subsequently "immune" to the blocking probe and is amplified at the same exponential rate as true mRNA derived cDNAs. Although PCR on genomic DNA with or without blocking probes (especially using the PNA probe) generated substantially differing amounts of product a total block of genomic *gfp* was not observed (data not shown). In order to asses the extend of inhibition we used a DNA fragment *(gfp* internal standard) (21) differing in length by an internal deletion from the transcribed part of the *gfp* sequence (between primers *gfp*mb1-Fx and gfpmb2-R). The internal standard DNA fragment has the exact same 3'end and 5'end as the expected *gfp* cDNA, and a mix of *gfp* internal standard DNA fragment and genomic DNA containing the *gfp* locus was used to mimic samples of mixed *gfp* cDNA and the homolog genomic DNA fragment. This is reasonable as the internal standard and the *gfp* sequence have the same primer target sites and PCR can be carried out at approximately similar amplification rates. Differing in size, *gfp* and *gfp* internal standard are readily separated on a gel and the ratio between PCR products resemble the ratio initially present in the sample. PCR was run with or without blocking probes using mixtures of *gfp* internal standard and genomic DNA derived from *P. putida* OUS82 UCB55 as template. The concentration of internal standard template was kept constant in all reactions while the concentration of genomic DNA was consistently halved over a series of 8 reactions. Results are shown in figure 4.

DNAB1 did not block the genomically derived *gfp* satisfactory, only moving the product equilibrium three steps to the left (figure 4, upper vs. lower panel).

Raising the blocking probe concentration resulted in suppression of both *gfp* and *gfp*- internal standard (data not shown). PNAB1, however, moved the equilibrium more than 6 steps to the left (figure 4, upper vs. middle panel). Raising the probe concentration did not result in increased suppression of *gfp* PCR product (data not shown). Due to the higher stability of a PNA/DNA duplex when compared to a DNA/DNA duplex, the event of primer invasion and subsequent amplification seems more likely using a blocking probe consisting of DNA than when using a PNA probe. The poor performance of the DNAB1 probe was therefore not surprising. Though the PNAB1 did not block the amplification of genomic *gfp* totally, it did suppress the amplification by approx. $2^6$ times. Hence less than 2% of the actual amount of contaminating genomic *gfp* was able to escape suppression and generate a PCR-product. Additionally, using PNAB1 seemed to enhance the overall PCR-reaction. Additions of non-specific oligonucleotides (tRNA) to (RT-) PCR reactions have been shown to increase product formation, presumably by binding loosely to excess primers during the initial amplification cycles (22). The enhanced PCR-performance after addition of PNAB1 is probably caused by a similar effect.

**Example 2**

**PNAB1 blocks the formation of genomic *gfp* derived RT-PCR product, while enhancing RT-PCR amplification of *gfp* mRNA using *P. putida* OUS82 UCB55 RNA samples as template.**

Duplicate samples of total RNA were extracted from approx. $10^7$ exponentially growing *P. putida* OUS82 UCB55 cells constitutively transcribing the *gfp* gene. One sample was reverse transcribed immediately and the other sample was treated with DNase prior to reverse transcription. Reverse transcription was performed with and without the RT-enzyme and the following PCR was performed with and without the PNA probe. Results are shown in figure 5.

The presence of DNA in the RNA extractions was confirmed (lane 7) and DNase treatment efficiently removed contaminating DNA (lane 3 and 4). A similar "removal" was observed using the PNA blocking probe (lane 8). By comparing lanes 1, 2 and 6 it seems evident that the PNA probe has a beneficial effect on the overall PCR performance (1 vs. 2) and that the DNase treatment has a negative effect on the quantity of mRNA-derived PCR product (2 vs. 6) (relative light intensities from products in lanes 1-8 respectively: 1.00; 1.42; 0.01; 0.06; 3.21; 2.72; 2.23; 0.15). These effects were confirmed when repeating the experiment and raising the number of PCR-cycles to 40. However when raising the number of PCR-cycles to 40 a visible band occurred in lane 8 (data not shown).

**Example 3**

**Quantitative RT-PCR estimates of *gfp* mRNA correlate to Gfp protein quantitations when using PNAB1 to block genomic gfp DNA.**

The PNA blocking probe was further tested using total RNA from the IPTG inducible strain *E. coli* SP308/pK3K10. Total RNA from $10^7$ cells was extracted in duplicates from 5 parallel cultures of E. *coli* SP308/pKJK10, grown exponentially for 2 hours in the presence of final IPTG concentrations of 0 μM, 100 μM, 500 μM, 1000μM and 2000μM, respectively. The cell density did not vary between cultures whereas fluorescence above background levels was measured in the cultures treated with 500 μM, 1000μM and 2000μM IPTG. Relative fluorescence intensities per cell at the time of sampling were 1.24; 1.29; 3.15; 7.95 and 16.30, respectively. Cell samples were immediately frozen in liquid nitrogen and kept at - 80°C until total RNA was extracted. All RNA samples were reverse transcribed immediately after extraction or treated with DNase and thereafter reverse transcribed. The following PCR was performed with or without blocking probe. Results are shown in figure 6.
Again the presence of DNA in the RNA extractions was confirmed and without DNase-treatment or blocking probe, no difference in the amount of RT-PCR product between different IPTG-treatments could be detected (lane 1-5) (relative light intensities were 1.00; 1.28; 0.71; 1.26; 0.96). DNase-treatment of samples efficiently removed DNA and in agreement with the FACS-data, RT-PCR product was detected in samples induced with 500, 1000 and 2000 μM IPTG (lane 6-10). However, quantitative estimates obtained by measuring light intensities of the ethidium bromide stained bands (-0.01; 0.00; 0.32; 0.52; 0.22) did not show the expected differential induction levels from different IPTG treatments. Adding PNAB1 to PCR reactions using the DNase treated samples as template, did again enhance the reaction generating increased amounts of product (lane 16-20)(relative light intensities of band were; -0.02; 0.00; 0.92; 0.60; 0.73).

When adding PNAB1 to the PCR step of the RT-PCR reaction using the non-DNase treated samples as template product of the expected size was detected in all treatments (lane 11-15) (relative light intensities were 0.43; 0.26; 0.71; 2.41; 2.66). This suggest that either PNAB1 did not block the genomic DNA or E. *coli* SP308/pKJK10 has a leaky expression of the *gfp* gene. Though no RT-PCR product could be generated after DNase treatment, the latter explanation seems the more likely since *E. coli* SP308/pKJK10 carries the *gfp*-gene on a multi-copy plasmid and the lac repressor is present as a single copy on the chromosome. That E. *coli* SP308/pKJK10 express *gfp* to some extend even in the absence of IPTG is also indicated by a lower average flourescense intensity per cell of the parental strain without the *gfp*-containing plasmid (data not shown). A small amount of messenger could, if present, have been degraded during the DNase-treatment, while giving rise to a RT-PCR product in the samples not treated with DNase. However, only small amounts of product were detected in the non-induced sample and product increased with the amount of IPTG added. An unexpected product of approx.1200 bp. was observed in all treatments. The presence of genomic DNA in the RT-PCR was apparently required for this product to arise, since this product was not present in the DNase treated samples. In a high stringency Southern analyses, a probe consisting of radioactively labelled *gfp* PCR-product was observed to hybridise to both bands, thus indicating the presence of the *gfp* sequence in both PCR fragments (data not shown). Furthermore, the amount of the 1200 bp product seems to be inversely related to the ratio between *gfp* mRNA and genomic *gfp* DNA templates. This indicates that the product could be related to the presence of excess primer in the initial PCR cycles. The expected size of a product formed by the simple duplication of the *gfp* sequence does not correlate with a product of size 1200bp. The origin of this PCR product, however, remains unclear.

**Example 4**

**PNAB2-reverse blocks the formation of rDNA PCR product in rRNA-targeted RT-PCR.**

rDNA targetted PCR using primers PRBA338F and 511R2, and blocking probe PNAB2- reverse was optimised using pre-primer-annealing temperatures between 56°C-65°C, and annealing temperatures between 45°C-55°C. Optimal PCR protocol (see above) resulted in near-optimal PCR-product formation in the absence of PNAB2-reverse and no visible product in the presence of PNAB2-reverse using genomic DNA from E. *coli* SP308/pKJK10 as template. Using purified PCR-product as template resulted in comparable amounts of PCR-products although PCR was slightly enhanced by the presence of PNAB2-reverse (data not shown). Thus the initial performance of this primer/probe pair was even more promising than PNAB1/*gfp*mb1-Fx. This probably reflect the freedom of choice on where to target the primer/probe pair, avoiding highly structured sequences and allowing a perfect match between probe and target sequence. As we did not get any visible PCR-product in the presence of the probe, construction of a new internal standard to test the extent of inhibition seemed inappropriate, and we chose to test the system directly on RNA samples.

Duplicate samples of total RNA was extracted from approx. $10^7$ exponentially growing and from approx. $10^7$ starving E. *coli* SP308/pKJK10 cells. One sample was reverse transcribed immediately and the other treated with DNase prior to reverse transcription. Reverse transcription was performed with and without the RT-enzyme and the following PCR was performed with and without the PNAB2-reverse probe. Results are shown in figure 7 (growing cells (a), and starving cells (b)).

Contaminating rDNA was present in both RNA extractions (lane 7 a, b) and DNase treatment efficiently removed contaminating DNA (lane 3 and 4 a, b). The PCR amplification of the contaminating rDNA was efficiently blocked by PNAB2-reverse (lane 8 a, b). Presence of rRNA in the starved cells was not detected after treatment of RNA samples with DNase. However the DNase-treatment resulted in a large "smear" of long PCR-products (lane 1, 2 b). Using PNAB2-reverse and omitting the DNase step showed that rRNA was present in small amounts in the samples from starved cells (lane 6 b). This experiment proves that using this technique has some advantages compared to conventional DNase treatment. Though using a reverse-targeted probe limits the use of the technique to a two-step RT-PCR procedure it seems evident that the increased freedom of choice in primer/probe design adds both efficiency and generality to the developed technique.

**Example 5**

**PNAinvA-reverse-1 blocks the formation of DNA based PCR product in quantification of mRNA from invA gene in *Salmonella thyphimorium***

rDNA targeted PCR using primers invAF2 and invAR1, and blocking probe PNAinvA-reverse-1 was optimised using pre-primer-annealing temperatures between 62°C-68°C, and annealing temperatures between 52-°C-67°C. Optimal PCR protocol (see above) resulted in near-optimal PCR-product formation in the absence of PNAinvA-reverse-1 and no visible product in the presence of PNAinvA-reverse-1 using genomic DNA from *S. thyphimorium* as template. In figure 5 Real Time RT-PCR amplification of cDNA from the *inv*A gene of S. *thyphimorium* without PCR signal from the corresponding DNA was accomplished using the PNA inhibitor probe PNAinvA-reverse-1 (compare lane 1 and lane 4). Comparable threshold values are seen for the Real Time analysis of the triplicate samples from DNase treated samples with or without the addition of PNA probe (Table 3). This result shows that the probe does not inhibit the PCR process when cDNA is the template.

Table 3:

| Threshold ($C_T$) values for Real Time PCR amplification of *Salmonella thyphimorium* InvA gene. For an explanation of the $C_T$-parameter see the previous detailed description of the invention in relation to the real-time PCR technology. | | | | | | |
|---|---|---|---|---|---|---|
| Threshold (Ct) values in Real time PCR | With PNA Rep. 1 | With PNA Rep. 2 | With PNA Rep. 3 | No PNA Rep. 1 | No PNA Rep. 2 | No PNA Rep. 3 |
| Direct nucleic acid extract (RNA and DNA) | N/A | N/A | N/A | 26.9 | 26.9 | 27.9 |
| Reverse transcribed after DNA'se treatment | 29.3 | 29.7 | 28.9 | 28.5 | 28.8 | 29.7 |
| Reverse transcribed no DNA'se treatment | 29.5 | 29.9 | 28.9 | 28.8 | 29.2 | 28.7 |

REFERENCES

1. Chen, J. & Griffiths, M.W. Detection of Salmonella and Shiga-like toxin-producing Escherichia coli using the magnetic capture hybridization polymerase chain reaction. Lett. Appl. Microbiol., 2001, Vol 32, pp. 7-11

2. Malonrny, B.; Hoorfar, J.; Bunge, C. & Helmuth, R. Multicenter validation of the analytical accuracy of Salmonella PCR: towards an international standard. Appl. Environ. Microbiol. 2003, Vol 69, pp. 290-296.

3. Hunziker, J. and Leumann, C. (1995) Nucleic Acid Analogues: Synthesis and Properties. In Modern Synthetic Methods, Vol. 7, pp. 331-417

4. Freier, S. M. & Altmann, K. H. The ups and downs of nucleic acid duplex stability: Structure-stability studies on chemically modified DNA:RNA duplexes. Nucl. Acid Res., 1997, 25, 4429-4443

5. Uhlmann, E. Recent advances in the medicinal chemistry of antisense oligonucleotides. Curr. Opinion in Drug & Development (2000, 3(2): 293-213.

6. del Mar Lleo M., S. Pierobon, M.C. Tafi, C. Signoretto, P. Canepari. 2000. mRNA detection by reverse transcription-PCR for monitoring viability over time in an Enterococcus faecalis viable but nonculturable population maintained in a laboratory microcosm. Appl Environ Microbiol 66: 4564-7.

7. Meckenstock R., P. Steinle, J.R. van der Meer, and M. Snozzi. 1998. Quantification of bacterial mRNA involved in degradation of 1,2,4-trichlorobenzene by Pseudomonas sp. Strain P51 from liquid culture and from river sediment by reverse transcriptase PCR (RT/PCR). FEMS Microbiol Lett 167: 123-129.

8. Wilson M.S., C. Bakermans, and E.L. Madsen. 1999. In situ, real-time catabolic gene expression: Extraction and characterization of naphtalene dioxygenase mRNA transcripts from groundwater. Appl Environ Microbiol 65: 80-87.

9. Alm E.V., and D.A. Stahl. 2000. Critical factors influencing the recovery and integrety of rRNA extracted from environmental samples: use of an optimized protocol to measure depth-related biomass distribution in freshwater sediments. J Microbiol Methods 40: 153-162

10. Tebbe C.C., W. Vahjen. 1993. Interference of humic acids and DNA extracted directly from soil in detection and transformation of recombinant DNA from bacteria and a yeast. Appl Environ Microbiol 59: 2657-65

11. Anonymous. 2000. Ambion Technotes Newsletter 7: 1-3

12. Ørum H., P. E. Nielsen, M. Engholm, R. H. Berg, O. Buchardt and C. Stanley. 1993. Single base pair mutation analysis by PNA directed PCR clamping. Nucleic Acids Res 21: 5332-5336.

13. Giesen U., W. Kleider, C. Berding, A. Geiger, H. Orum, P.E. Nielsen. 1998. A formula for thermal stability (Tm) prediction of PNA/DNA duplexes. Nucleic Acids Res 26: 5004-6.

**14.** Cochet O., E. Martin, W.H. Fridman., J.L. Teillaud. 1999. Selective PCR amplification of functional immunoglobulin light chain from hybridoma containing the aberrant MOPC 21-derived V kappa by PNA-mediated PCR clamping. Biotechniques 26: 818-20, 822.

15. Thiede C., E. Bayerdorffer, R. Blasczyk, B. Wittig, A. Neubauer. 1996. Simple and sensitive detection of mutations in the ras proto-oncogenes using PNA-mediated PCR clamping. Nucleic Acids Res 24: :983-4

16. von Wintzingerode F., O. Landt, A. Ehrlich, U.B. Gobel. 2000. Peptide nucleic acid-mediated PCR clamping as a useful supplement in the determination of microbial diversity. Appl Environ Microbiol 66: 549-57.

17. Sengeløv G., K.J. Kristensen, A.H. Sørensen, N. Kroer, S.J. Sørensen. 2001. Effect of genomic location on horizontal transfer of a recombinant gene cassette between pseudomonas strains in the rhizosphere and sphermosphere of barley seedlings. Curr Microbiol 42: 160-167.

18. Tolker-Nielsen T., U.C. Brinch, P.C. Ragas, J.B. Andersen, C.S. Jacobsen, S. Molin. 2000. Development and dynamics of Pseudomonas sp. biofilms. J Bacteriol 182: 6482-9.

17. Andersen J.B., C. Sternberg, L.K. Poulsen, S.P. Bjorn, M. Givskov, S. Molin. 1998. New unstable variants of green fluorescent protein for studies of transient gene expression in bacteria. Appl Environ Microbiol 1998 64: 2240-6

18. Lanzer M., and H. Bujard. 1988. Promoters largely determine the efficiency of repressor action. Proc Natl Acad Sci U S A 85: 8973-7.

19. Kiyohara H., N. Takizawa, and K. Nagao. 1992. Natural distribution of bacteria metabolizing many kinds of polycyclic aromatic hydrocarbons. J Ferment Bioeng 74: 49-51

20. Kiyohara H., S. Torigoe, N. Kaida, T. Asaki, T. Iida, H. Hayashi, N. Takizawa. 1994. Cloning and characterization of a chromosomal gene cluster, pah, that encodes the upper pathway for phenanthrene and naphthalene utilization by Pseudomonas putida OUS82. J Bacteriol 176. 2439-43

21. Bender M., U.C. Brinch, and C.S. Jacobsen. 2003 Development and application of a quantitative PCR technique targeting gfp-tagged flagella mutants of Pseudomonas putida OUS82 in soil and sediment columns. In prep**.**

22. Sturzenbaum S.R. 1999. Transfer RNA reduces the formation of primer artifacts during quantitative PCR. Biotechniques 27: 50-2.

23. WO-A-01/20041

## Claims

**1.** A method for detecting the presence or absence of at least one target nucleic acid sequence in a sample that further contains a nucleic acid molecule comprising a sequence corresponding to the target nucleic acid sequence, the method comprising the step of contacting the sample with a first extendable primer and at least one nucleic acid probe that is capable of selectively binding to a continuum of at least a part of the nucleic acid molecule corresponding to the target nucleic acid sequence and a part of the nucleic acid molecule adjacent to said corresponding sequence, wherein said target nucleic acid sequence and its complementary strand is amplified in at least one thermocycle comprising the steps of primer annealing, primer extension and denaturation, and wherein binding of the at least one nucleic acid probe to the nucleic acid molecule comprising a sequence corresponding to the target nucleic acid sequence prevents annealing of said extendable primer hereto and extension of the primer, without affecting annealing of the extendable primer to the target nucleic acid sequence.

2. A method according to claim 1 wherein the continuum of at least a part of the nucleic acid molecule corresponding to the target nucleic acid sequence and a part of the nucleic add molecule adjacent to said corresponding sequence comprises a transcription initiation site and its upstream sequence.

3. A method according to any of claims 1 or 2, wherein the nucleic acid probe comprises a first part, which will anneal to a 3' region within said part of the nucleic acid molecule corresponding to the target nucleic acid sequence under stringent conditions, and a second part, which will anneal to a neighbouring sequence of the nucleic acid molecule under stringent conditions.

4. A method according to claim 3, wherein said first part of the nucleic acid probe is shorter than the first extendable primer.

5. A method according to any of claims 1 to 4, wherein the $T_m$-value for the expected hybrid of the nucleic acid probe and the nucleic acid molecule comprising a sequence corresponding to the target nucleic acid sequence is higher than the $T_m$-value for the expected hybrid of the first extendable primer and the target nucleic acid sequence.

6. A method according to any of the preceding claims, wherein annealing of the nucleic acid probe and annealing of the first extendable primer occur in two successive steps and the step in which annealing of the nucleic acid probe occurs precedes the step in which annealing of the first extendable primer occurs.

7. A method according to any of claims 1 to 6, wherein the nucleic acid probe is a nucleic acid analog.

8. A method according to any of claims 1 to 7, wherein the target nucleic acid sequence is derived from an RNA sequence.

9. A method according to any of claims 1 to 8, wherein the target nucleic acid sequence is derived from a ribosomal RNA.

10. A method according to any of the preceding claims, wherein the target nucleic acid is identical to a nucleic acid sequence, which is specific for one or more microbial species, or its complementary sequence.

11. A method according to claim 10, wherein the target nucleic acid sequence is identical to a nucleic acid sequence which is derived from the invA gene of *Salmonella Spp*., or identical to the complementary sequence of said derived sequence.

12. A method according to any of the preceding claims, wherein the nucleic acid molecule comprising a sequence corresponding to the target nucleic acid sequence is a genomic DNA molecule.

13. A method according to any of the preceding claims, wherein the nucleic add molecule comprising a sequence corresponding to the target nucleic acid sequence is a nucleic acid molecule comprising a sequence, which is complementary to the target nucleic add sequence.

14. A method according to any of claims 1 to 13 further comprising contacting the sample with a second extendable primer which will anneal to a part of the complementary strand of said target nucleic acid sequence under stringent conditions and detecting the target nucleic acid sequence subsequent to its amplification.

15. A method according to any of claims 1 to 14, wherein said sample is obtained from a food source.

16. A method according to any of claims 1 to 14, wherein said sample is obtained from a source of drinking water.

17. A method according to any of claims 10 and 12 to 16, wherein the microbial species is a virus.

18. A method according to any of the preceding claims further comprising the step of contacting the sample with at least one second nucleic acid probe capable of binding to a continuum of at least a part of the nucleic acid molecule corresponding to a sequence complementary to the target nucleic acid sequence and a part of the nucleic acid molecule adjacent to said corresponding sequence.

## Patentansprüche

1. Verfahren zur Erkennung der Anwesenheit oder der Abwesenheit mindestens einer Zielnukleinsäuresequenz in einer Probe, die weiterhin ein Nukleinsäuremolekül enthält, das eine Sequenz umfasst, die der Zielnukleinsäuresequenz entspricht, wobei das Verfahren den Schritt umfasst, Kontaktieren der Probe mit einem ersten verlängerbaren Primer und mindestens einer Nukleinsäureprobe, die in der Lage ist, an ein Kontinuum von mindestens einem Teil des Nukleinsäuremoleküls, der der Zielnukleinsäuresequenz entspricht, und einem Teil des Nukleinsäuremoleküls, der an der entsprechenden Sequenz angrenzt, selektiv zu binden, worin die Zielnukleinsäuresequenz und deren komplementärer Strang in mindestens einem Thermozyklus amplifiziert wird, der die Schritte Primer-Anlagerung, Primer-Verlängerung und Denaturierung umfasst, und worin das Binden der zumindest einen Nukleinsäureprobe an das Nukleinsäuremolekül, das eine Sequenz umfasst, die der Zielnukleinsäuresequenz entspricht, die Anlagerung des verlängerbaren Primers hierzu und die Verlängerung des Primers verhindert, ohne die Anlagerung des verlängerbaren Primers zur Zielnukleinsäuresequenz zu beeinflussen.

2. Verfahren nach Anspruch 1, worin das Kontinuum von mindestens einem Teil des Nukleinsäuremoleküls, das der Zielnukleinsäuresequenz entspricht und einem Teil der Nukleinsäuremoleküls, das an der entsprechenden Sequenz angrenzt, einen Transkriptionsstartpunkt und dessen Stromaufwärtssequenz umfasst.

3. Verfahren nach irgendeinem der Ansprüche 1 oder 2, worin die Nukleinsäureprobe einen ersten Teil umfasst, der unter stringenten Bedingungen an einen 3' Bereich innerhalb des Teils des Nukleinsäuremoleküls anlagert, das der Zielnukleinsäuresequenz entspricht, und einen zweiten Teil umfasst, der unter stringenten Bedingungen an eine benachbarte Sequenz des Nukleinsäuremoleküls anlagert.

4. Verfahren nach Anspruch 3, worin der erste Teil der Nukleinsäureprobe kürzer als der erste verlängerbare Primer ist.

5. Verfahren nach irgendeinem der Ansprüche 1 bis 4, worin der $T_m$-Wert für das erwarteten Hybrid der Nukleinsäureprobe und des Nukleinsäuremoleküls, die eine Sequenz umfasst, die der Zielnukleinsäuresequenz entspricht, höher als der $T_m$-Wert für das erwartete Hybrid des ersten verlängerbaren Primers und der Zielnukleinsäuresequenz ist.

6. Verfahren nach irgendeinem der vorhergehenden Ansprüche, worin eine Anlagerung der Nukleinsäureprobe und eine Anlagerung des ersten verlängerbaren Primers in zwei nacheinander folgenden Schritten erfolgt und der Schritt, bei dem eine Anlagerung der Nukleinsäureprobe erfolgt vor dem Schritt, bei dem die Anlagerung des ersten verlängerbaren Primers erfolgt, vorgeht.

7. Verfahren nach irgendeinem der Ansprüche 1 bis 6, worin die Nukleinsäureprobe ein Nukleinsäureanalogon ist.

8. Verfahren nach irgendeinem der Ansprüche 1 bis 7, worin die Zielnukleinsäureseqenz von einer RNA-Sequenz abgeleitet ist.

9. Verfahren nach irgendeinem der Ansprüche 1 bis 8, worin die Zielnukleinsäureseqenz von einer ribosomalen RNA abgeleitet ist.

10. Verfahren nach irgendeinem der vorhergehenden Ansprüche, worin die Zielnukleinsäure mit einer Nukleinsäuresequenz identisch ist, die für eine oder mehrere mikrobielle Spezies spezifisch ist, oder dessen komplementäre Sequenz.

11. Verfahren nach Anspruch 10, worin die Zielnukleinsäuresequenz mit einer Nukleinsäuresequenz identisch ist, die aus dem invA-Gen von *Salmonella Spp.* abgeleitet ist, oder mit der komplementären Sequenz der abgeleiteten Sequenz identisch ist.

12. Verfahren nach irgendeinem der vorhergehenden Ansprüche, worin das Nukleinsäuremolekül, das eine Sequenz umfasst, die der Zielnukleinsäuresequenz entspricht, ein genomisches DNA-Molekül ist.

13. Verfahren nach irgendeinem der vorhergehenden Ansprüche, worin das Nukleinsäuremolekül, das eine Sequenz umfasst, die der Zielnukleinsäuresequenz entspricht, ein Nukleinsäuremolekül ist, das eine Sequenz umfasst, die mit der Zielnukleinsäuresequenz komplementär ist.

14. Verfahren nach irgendeinem der Ansprüche 1 bis 13, das weiterhin umfasst, Kontaktieren der Probe mit einem zweiten verlängerbaren Primer, der unter stringenten Bedingungen an einen Teil des komplementären Strangs der Zielnukleinsäuresequenz anlagert und Erkennung der Zielnukleinsäuresequenz nach seiner Amplifikation.

15. Verfahren nach irgendeinem der Ansprüche 1 bis 14, worin die Probe von einer Lebensmittelquelle erhalten ist.

16. Verfahren nach irgendeinem der Ansprüche 1 bis 14, worin die Probe von einer Trinkwasserquelle erhalten ist.

17. Verfahren nach irgendeinem der Ansprüche 10 und 12 bis 16, worin die mikrobielle Spezies ein Virus ist.

18. Verfahren nach irgendeinem der vorhergehenden Ansprüche, weiterhin umfassend den Schritt, Kontaktieren der Probe mit mindestens einer zweiten Nukleinsäureprobe, die in der Lage ist, an ein Kontinuum von mindestens einem Teil des Nukleinsäuremoleküls, das einer Sequenz entspricht, die mit der Zielnukleinsäuresequenz komplementär ist, und einem Teil des Nukleinsäuremoleküls, der an der korrespondierenden Sequenz angrenzt, zu binden.

**Revendications**

1. Procédé pour détecter la présence ou l'absence d'au moins une séquence d'acide nucléique cible dans un échantillon qui contient en outre une molécule d'acide nucléique comprenant une séquence correspondant à la séquence d'acide nucléique cible, le procédé comprenant une étape consistant à mettre l'échantillon en contact avec une première amorce extensible et au moins une sonde nucléique qui est capable de se lier de façon sélective à un continuum d'au moins une partie de la molécule d'acide nucléique correspondant à la séquence d'acide nucléique cible et une partie de la molécule d'acide nucléique adjacente à ladite séquence correspondante, dans lequel ladite séquence d'acide nucléique cible et son brin complémentaire sont amplifiés dans au moins un thermocycle comprenant les étapes d'hybridation d'amorce, d'extension d'amorce et de dénaturation, et dans lequel la liaison de la au moins une sonde nucléique avec la molécule d'acide nucléique comprenant une séquence correspondant à la séquence d'acide nucléique cible empêche l'hybridation de ladite amorce extensible jusque-là et l'extension de l'amorce, sans affecter l'hybridation de l'amorce extensible jusqu'à la séquence d'acide nucléique cible.

2. Procédé selon la revendication 1, dans lequel le continuum d'au moins une partie de la molécule d'acide nucléique correspondant à la séquence d'acide nucléique cible et une partie de la molécule d'acide nucléique adjacente à ladite séquence correspondante comprend un site d'initiation de transcription et la séquence amont de celui-ci.

3. Procédé selon l'une des revendications 1 ou 2, dans lequel la sonde nucléique comprend une première partie qui s'hybridera avec une région 3' dans ladite partie de la molécule d'acide nucléique correspondant à la séquence d'acide nucléique cible dans des conditions stringentes, et une seconde partie qui s'hybridera avec la séquence voisine de la molécule d'acide nucléique dans des conditions stringentes.

4. Procédé selon la revendication 3, dans lequel ladite première partie de la sonde nucléique est plus courte que la première amorce extensible.

5. Procédé selon l'une des revendications 1 à 4, dans lequel la valeur $T_m$ de l'hybride attendu entre la sonde nucléique et la molécule d'acide nucléique comprenant une séquence correspondant à la séquence d'acide nucléique cible est supérieure à la valeur $T_m$ de l'hybride attendu entre la première amorce extensible et la séquence d'acide nucléique cible.

6. Procédé selon l'une des revendications précédentes, dans lequel l'hybridation de la sonde nucléique et l'hybridation de la première amorce extensible ont lieu en deux étapes successives, et l'étape dans laquelle a lieu l'hybridation de la sonde nucléique précède l'étape dans laquelle a lieu l'hybridation de la première amorce extensible.

7. Procédé selon l'une des revendications 1 à 6, dans lequel la sonde nucléique est un analogue d'acide nucléique.

8. Procédé selon l'une des revendications 1 à 7, dans lequel la séquence d'acide nucléique cible est dérivée d'une séquence d'ARN.

9. Procédé selon l'une des revendications 1 à 8, dans lequel la séquence d'acide nucléique cible est dérivée d'un ARN ribosomal.

**10.** Procédé selon l'une des revendications précédentes, dans lequel l'acide nucléique cible est identique à une séquence d'acide nucléique qui est spécifique à une ou plusieurs espèces microbiennes, ou à la séquence complémentaire à celle-ci.

**11.** Procédé selon la revendication 10, dans lequel la séquence est identique à une séquence d'acide nucléique qui est dérivée du gène invA de *Salmonella Spp.*, ou bien identique à la séquence complémentaire à ladite séquence dérivée.

**12.** Procédé selon l'une des revendications précédentes, dans lequel la molécule d'acide nucléique comprenant une séquence correspondant à la séquence d'acide nucléique cible est une molécule d'ADN génomique.

**13.** Procédé selon l'une des revendications précédentes, dans lequel la molécule d'acide nucléique comprenant une séquence correspondant à la séquence d'acide nucléique cible est une molécule d'acide nucléique comprenant une séquence qui est complémentaire à la séquence d'acide nucléique cible.

**14.** Procédé selon l'une des revendications 1 à 13 comprenant en outre la mise en contact de l'échantillon avec une deuxième amorce extensible qui s'hybridera avec une partie du brin complémentaire de ladite séquence d'acide nucléique cible dans des conditions stringentes, et la détection de la séquence d'acide nucléique cible après son amplification.

**15.** Procédé selon l'une des revendications 1 à 14, dans lequel ledit échantillon est obtenu à partir d'une source alimentaire.

**16.** Procédé selon l'une des revendications 1 à 14, dans lequel ledit échantillon est obtenu à partir d'une source d'eau potable.

**17.** Procédé selon l'une des revendications 10 et 12 à 16, dans lequel l'espèce microbienne est un virus.

**18.** Procédé selon l'une des revendications précédentes comprenant en outre l'étape de mettre l'échantillon en contact avec une seconde sonde nucléique capable de se lier à un continuum d'au moins une partie de la molécule d'acide nucléique correspondant à une séquence complémentaire à la séquence d'acide nucléique cible et une partie de la molécule d'acide nucléique adjacente à ladite séquence correspondante.

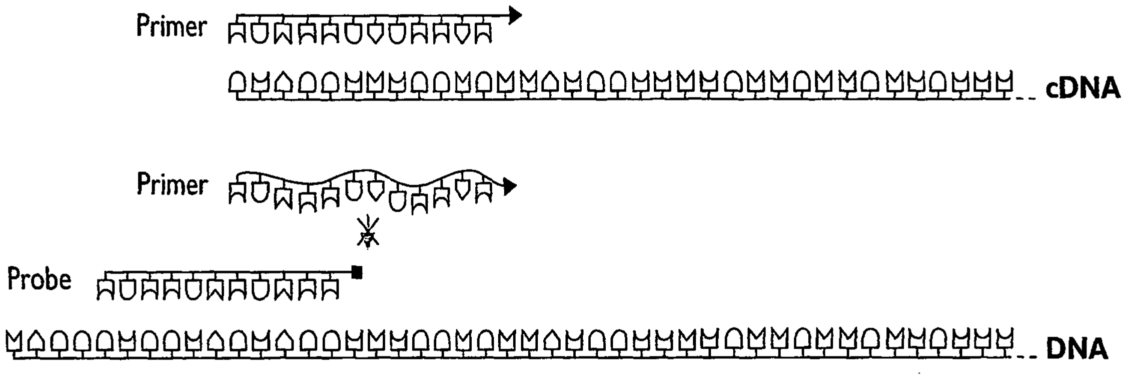


Fig. 1

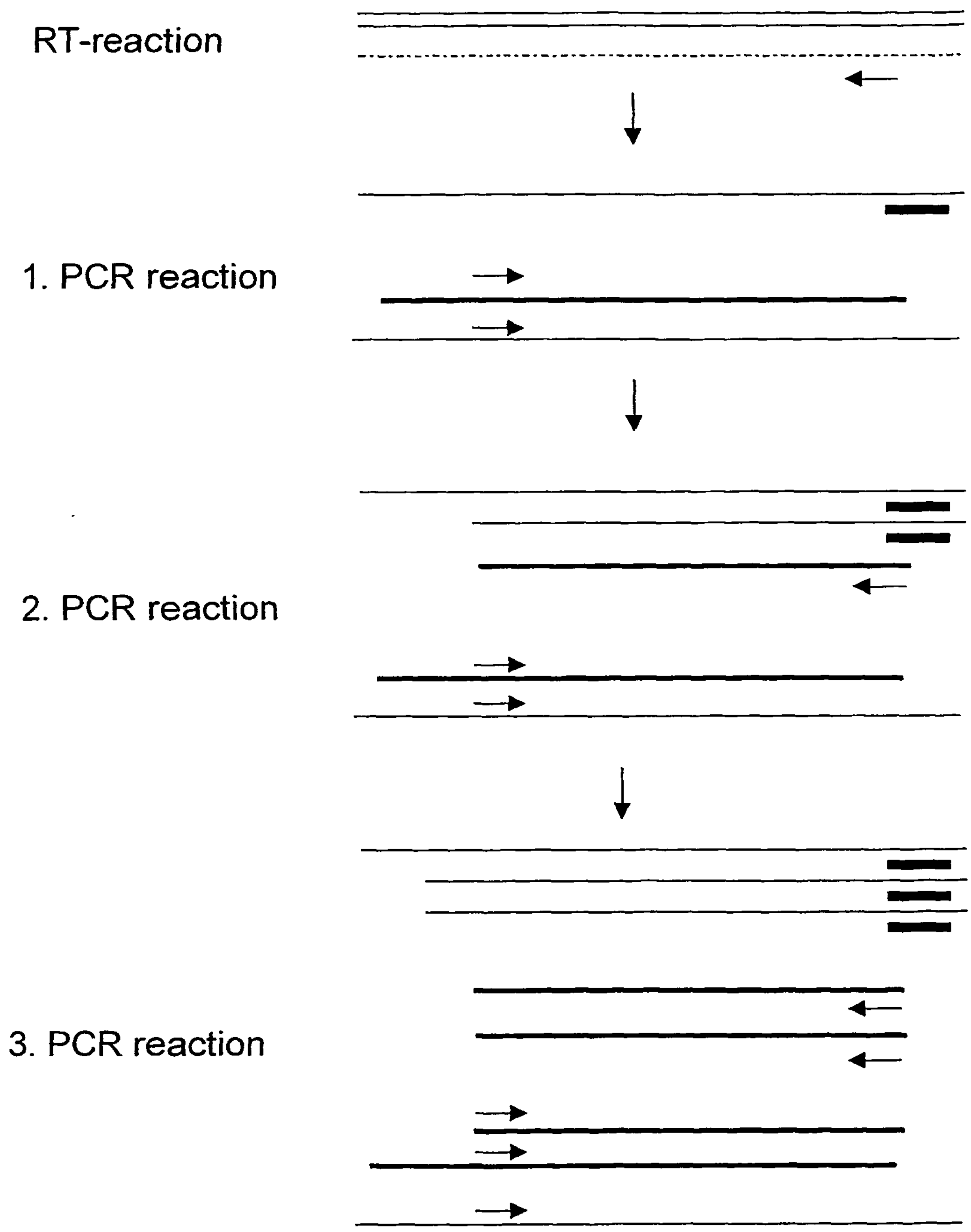


Fig. 2

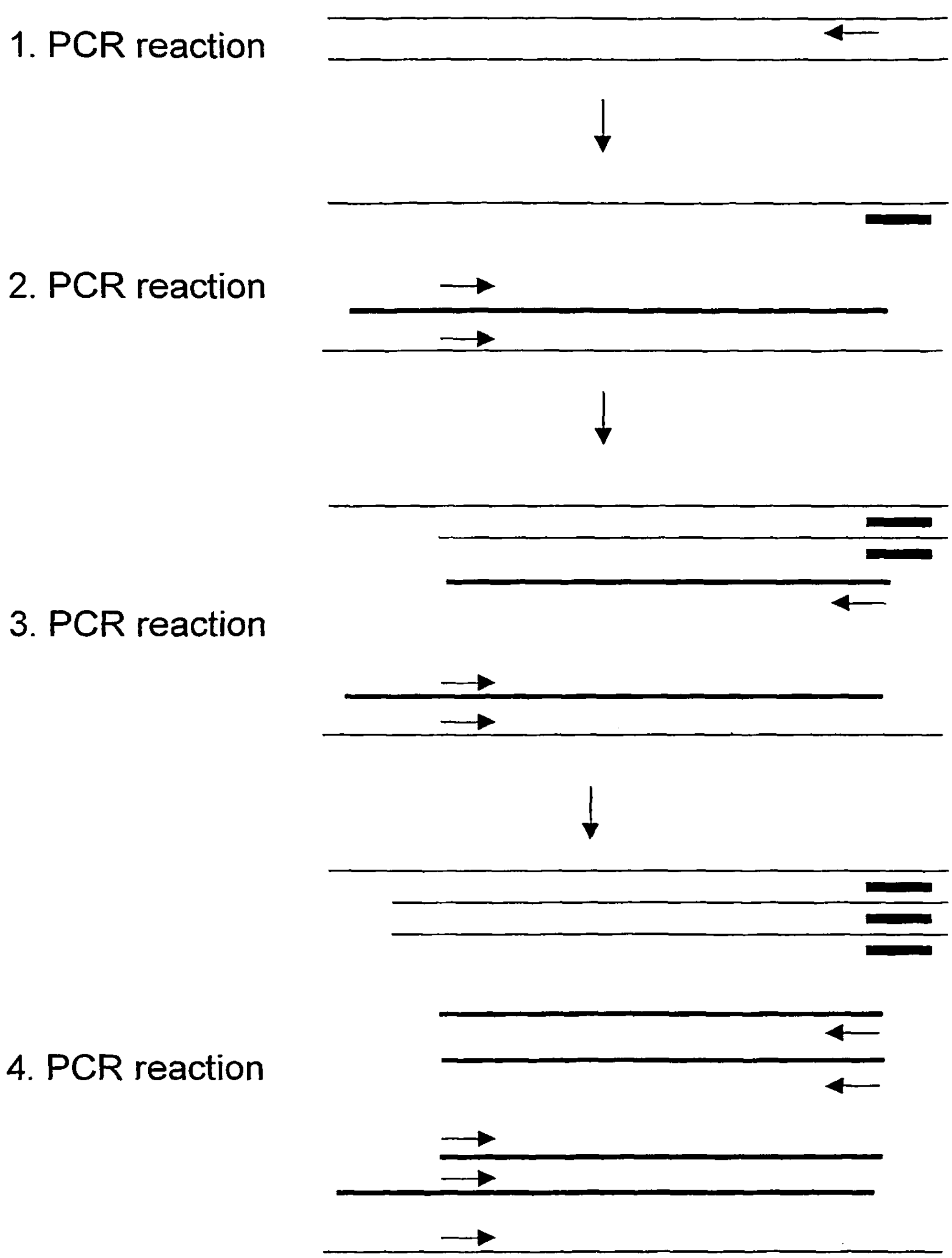


Fig. 3

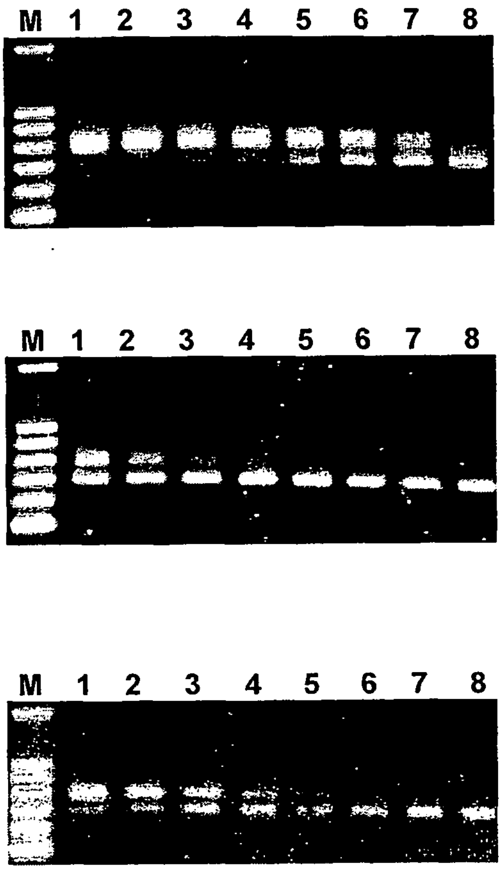


Fig. 4

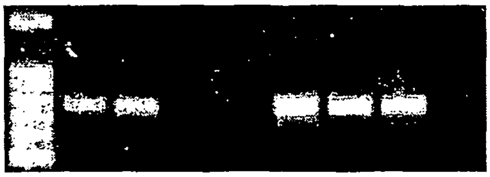


Fig. 5

M 1 2 3 4 5 6 7 8 9 10 11 12 13 14 15 16 17 18 19 20

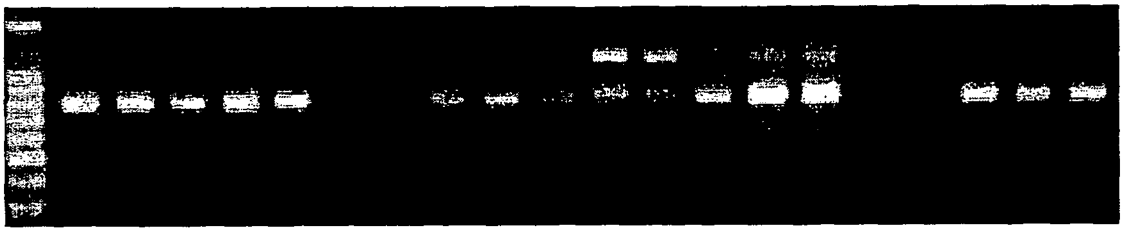

Fig.6

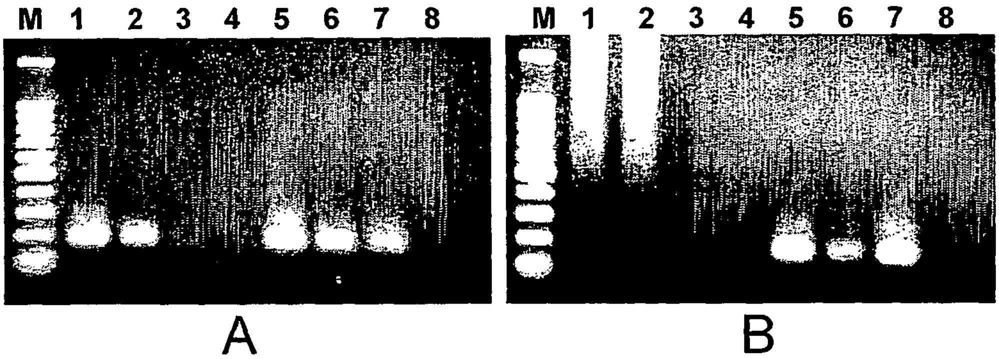


Fig. 7

1 2 3 4 5 6 M -

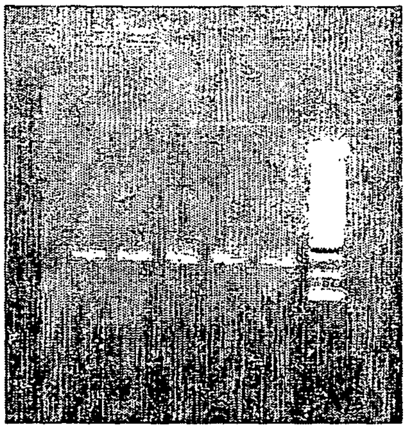

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0120041 A
- DK 20040004480 W


### Non-patent literature cited in the description


- **CHEN, J. ; GRIFFITHS, M.W.** Detection of Salmonella and Shiga-like toxin-producing Escherichia coli using the magnetic capture hybridization polymerase chain reaction. *Lett. Appl. Microbiol.,* 2001, vol. 32, 7-11
- **MALONRNY, B. ; HOORFAR, J. ; BUNGE, C. ; HELMUTH, R.** Multicenter validation of the analytical accuracy of Salmonella PCR: towards an international standard. *Appl. Environ. Microbiol.,* 2003, vol. 69, 290-296
- **HUNZIKER, J. ; LEUMANN, C.** Nucleic Acid Analogues: Synthesis and Properties. *Modern Synthetic Methods,* 1995, vol. 7, 331-417
- **FREIER, S. M. ; ALTMANN, K. H.** The ups and downs of nucleic acid duplex stability: Structure-stability studies on chemically modified DNA:RNA duplexes. *Nucl. Acid Res.,* 1997, vol. 25, 4429-4443
- **UHLMANN, E.** Recent advances in the medicinal chemistry of antisense oligonucleotides. *Curr. Opinion in Drug & Development,* 2000, vol. 3 (2), 293-213
- **DEL MAR LLEO M. ; S. PIEROBON ; M.C. TAFI ; C. SIGNORETTO ; P. CANEPARI.** mRNA detection by reverse transcription-PCR for monitoring viability over time in an Enterococcus faecalis viable but nonculturable population maintained in a laboratory microcosm. *Appl Environ Microbiol,* 2000, vol. 66, 4564-7
- **MECKENSTOCK R. ; P. STEINLE ; J.R. VAN DER MEER ; M. SNOZZI.** Quantification of bacterial mRNA involved in degradation of 1,2,4-trichlorobenzene by Pseudomonas sp. Strain P51 from liquid culture and from river sediment by reverse transcriptase PCR (RT/PCR). *FEMS Microbiol Lett,* 1998, vol. 167, 123-129
- **WILSON M.S. ; C. BAKERMANS ; E.L. MADSEN.** In situ, real-time catabolic gene expression: Extraction and characterization of naphtalene dioxygenase mRNA transcripts from groundwater. *Appl Environ Microbiol,* 1999, vol. 65, 80-87
- **ALM E.V. ; D.A. STAHL.** Critical factors influencing the recovery and integrety of rRNA extracted from environmental samples: use of an optimized protocol to measure depth-related biomass distribution in freshwater sediments. *J Microbiol Methods,* 2000, vol. 40, 153-162
- **TEBBE C.C. ; W. VAHJEN.** Interference of humic acids and DNA extracted directly from soil in detection and transformation of recombinant DNA from bacteria and a yeast. *Appl Environ Microbiol,* 1993, vol. 59, 2657-65
- **ANONYMOUS.** *Ambion Technotes Newsletter,* 2000, vol. 7, 1-3
- **ØRUM H. ; P. E. NIELSEN ; M. ENGHOLM ; R. H. BERG ; O. BUCHARDT ; C. STANLEY.** Single base pair mutation analysis by PNA directed PCR clamping. *Nucleic Acids Res,* 1993, vol. 21, 5332-5336
- **GIESEN U. ; W. KLEIDER ; C. BERDING ; A. GEIGER ; H. ORUM ; P.E. NIELSEN.** A formula for thermal stability (Tm) prediction of PNA/DNA duplexes. *Nucleic Acids Res,* 1998, vol. 26, 5004-6
- **COCHET O. ; E. MARTIN ; W.H. FRIDMAN. ; J.L. TEILLAUD.** Selective PCR amplification of functional immunoglobulin light chain from hybridoma containing the aberrant MOPC 21-derived V kappa by PNA-mediated PCR clamping. *Biotechniques,* 1999, vol. 26, 818-20 822
- **THIEDE C. ; E. BAYERDORFFER ; R. BLASCZYK ; B. WITTIG ; A. NEUBAUER.** Simple and sensitive detection of mutations in the ras proto-oncogenes using PNA-mediated PCR clamping. *Nucleic Acids Res,* 1996, vol. 24, 983-4
- **VON WINTZINGERODE F. ; O. LANDT ; A. EHRLICH ; U.B. GOBEL.** Peptide nucleic acid-mediated PCR clamping as a useful supplement in the determination of microbial diversity. *Appl Environ Microbiol,* 2000, vol. 66, 549-57

- **SENGELØV G. ; K.J. KRISTENSEN ; A.H. SØRENSEN ; N. KROER ; S.J. SØRENSEN.** Effect of genomic location on horizontal transfer of a recombinant gene cassette between pseudomonas strains in the rhizosphere and sphermosphere of barley seedlings. *Curr Microbiol,* 2001, vol. 42, 160-167
- **TOLKER-NIELSEN T. ; U.C. BRINCH ; P.C. RAGAS ; J.B. ANDERSEN ; C.S. JACOBSEN ; S. MOLIN.** Development and dynamics of Pseudomonas sp. biofilms. *J Bacteriol,* 2000, vol. 182, 6482-9
- **ANDERSEN J.B. ; C. STERNBERG ; L.K. POULSEN ; S.P. BJORN ; M. GIVSKOV ; S. MOLIN.** New unstable variants of green fluorescent protein for studies of transient gene expression in bacteria. *Appl Environ Microbiol 1998,* 1998, vol. 64, 2240-6
- **LANZER M. ; H. BUJARD.** Promoters largely determine the efficiency of repressor action. *Proc Natl Acad Sci U S A,* 1988, vol. 85, 8973-7
- **KIYOHARA H. ; N. TAKIZAWA ; K. NAGAO.** Natural distribution of bacteria metabolizing many kinds of polycyclic aromatic hydrocarbons. *J Ferment Bioeng,* 1992, vol. 74, 49-51
- **KIYOHARA H. ; S. TORIGOE ; N. KAIDA ; T. ASAKI ; T. IIDA ; H. HAYASHI ; N. TAKIZAWA.** Cloning and characterization of a chromosomal gene cluster, pah, that encodes the upper pathway for phenanthrene and naphthalene utilization by Pseudomonas putida OUS82. *J Bacteriol,* 1994, vol. 176, 2439-43
- **BENDER M. ; U.C. BRINCH ; C.S. JACOBSEN.** *Development and application of a quantitative PCR technique targeting gfp-tagged flagella mutants of Pseudomonas putida OUS82 in soil and sediment columns.,* 2003
- **STURZENBAUM S.R.** Transfer RNA reduces the formation of primer artifacts during quantitative PCR. *Biotechniques,* 1999, vol. 27, 50-2